(19) Europäisches Patentamt
European Patent Office
Office européen des brevets

(11) Publication number: **0 032 009**
**A1**

# (12) EUROPEAN PATENT APPLICATION

(21) Application number: 80304471.8

(22) Date of filing: 11.12.80

(51) Int. Cl.³: A 61 K 37/02
C 07 C 103/52, C 12 P 21/04
//(C12P21/04, C12R1/045, 1/62)

(30) Priority: 13.12.79 US 103149
13.12.79 US 103015
13.12.79 US 103147
13.12.79 US 103131
13.12.79 US 103148
25.08.80 US 181030
25.08.80 US 181040
25.08.80 US 182248
25.08.80 US 181435
25.08.80 US 181436

(43) Date of publication of application:
15.07.81 Bulletin 81/28

(84) Designated Contracting States:
DE GB LU NL SE

(71) Applicant: ELI LILLY AND COMPANY
307, East McCarty Street
Indianapolis Indiana 46206(US)

(72) Inventor: Debono, Manuel
5257, Hinesley Avenue
Indianapolis Indiana(US)

(74) Representative: Crowther, Terence Roger
Erl Wood Manor
Windlesham Surrey GU20 6PH(GB)

(54) **Derivatives of cyclic peptide nuclei.**

(57) Compounds of the formula

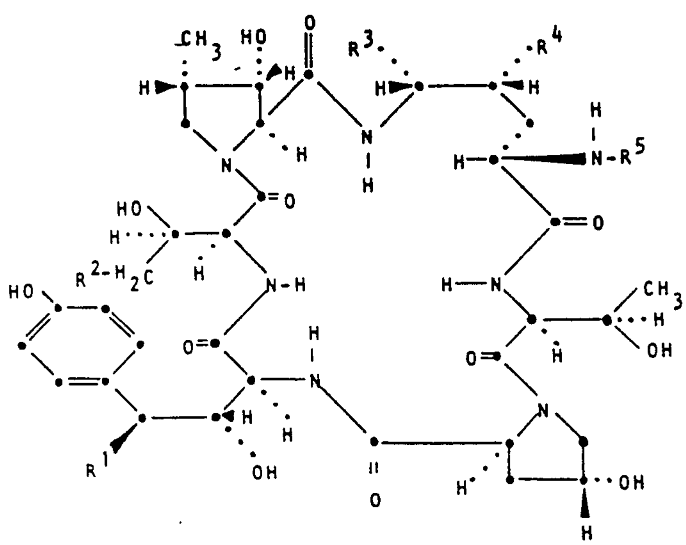

./...

EP 0 032 009 A1

wherein $R^1$ is H or OH and;

when $R^1$ is H, $R^2$ is H and $R^3$ and $R^4$ are both H or both OH,

and

when $R^1$ is OH, $R^2$ is H, $R^3$ is OH or $C_1$-$C_6$ alkyloxy and $R^4$ is OH, or $R^2$ is -CO-NH$_2$ and $R^3$ and $R^4$ are both OH;

$R^5$ is an N-alkanoyl amino acyl group of the formula $-W-\overset{O}{\overset{\|}{C}}-R^6$ wherein:

W is a divalent aminoacyl radical of the formula:

(a) $-\overset{O}{\overset{\|}{C}}-A-NH-$

wherein A is $C_1$-$C_{10}$ alkylene or $C_5$-$C_6$ cycloalkylene;

(b) $-\overset{O}{\overset{\|}{C}}-\overset{R^7}{CH}-NH-$

wherein $R^7$ is hydroxymethyl, hydroxyethyl, mercaptomethyl, mercaptoethyl, methylthioethyl, 2-thienyl, 3-indole-methy, phenyl, benzyl, or substituted phenyl or substituted benzyl in which the benzene ring thereof is substituted with chloro, bromo, iodo, nitro, $C_1$-$C_3$ alkyl, hydroxyl, $C_1$-$C_3$ alkylthio, carbamyl, or $C_1$-$C_3$ alkylcarbamyl;

(c)

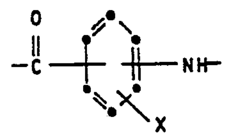

wherein X is hydrogen chloro, bromo, iodo, nitro, $C_1$-$C_3$ alkyl, hydroxy, $C_1$-$C_3$ alkoxy, mercapto, $C_1$-$C_3$ alkylthio, carbamyl, or $C_1$-$C_3$ alkylcarbamyl;

(d)

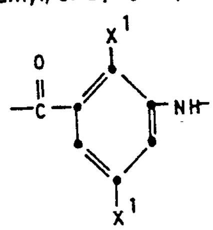

or

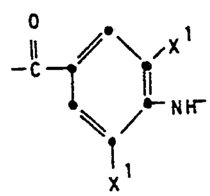

wherein $X^1$ is chloro, bromo, or iodo;

(e)

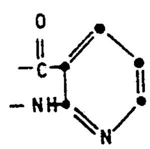

or

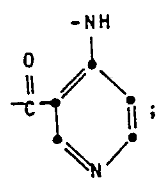

; or

(f)

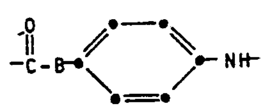

wherein B is a divalent radical of the formula: $-(CH_2)_n-$, wherein n is an integer from 1 to 3; -CH=CH-; -CH=CH-CH$_2$-; or

$-\overset{O}{\overset{\|}{C}}NHCH_2-$

and $R^6$ is $C_1$-$C_{17}$ alkyl or $C_2$-$C_{17}$ alkenyl which have antifungal activity.

# DERIVATIVES OF CYCLIC PEPTIDE NUCLEI

This invention relates to novel semi-synthetic antifungal compounds which are prepared by the acylation of cyclic peptide nuclei produced by the enzymatic deacylation of a corresponding cyclic peptide antibiotic.

The cyclic peptide antibiotic is an antifungal compound having the general formula:

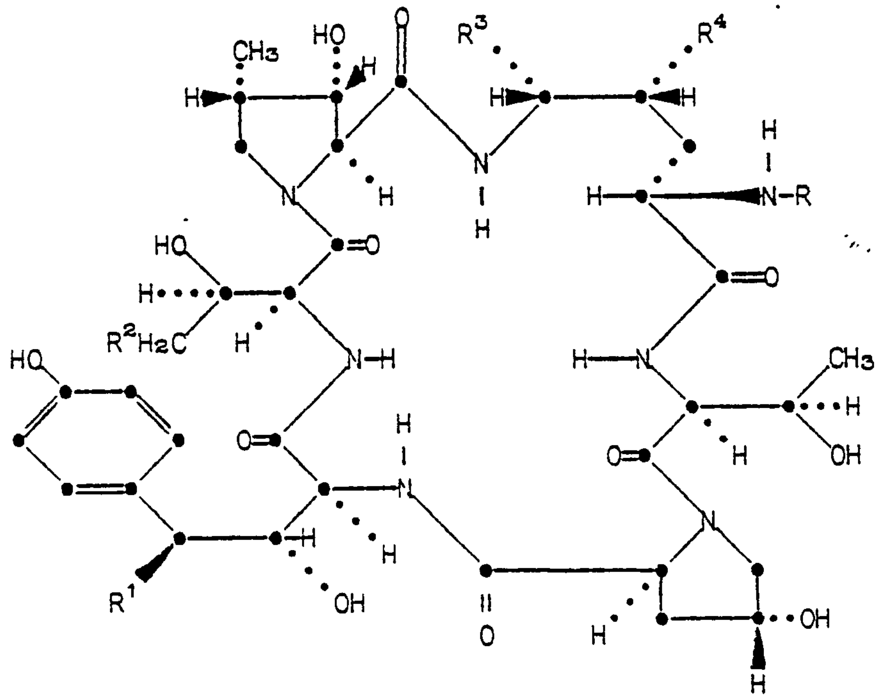

I

wherein R, $R^1$, $R^2$, $R^3$ and $R^4$ are defined herein below. Throughout this application, the cyclic peptide formulas, such as formula I, assume that the amino acids represented are in the L-configuration.

The A-30912 factors A, B, D and H are cyclic peptide antibiotics of the general formula I wherein R is the linoleoyl group [<u>cis</u>,<u>cis</u> $CH_3(CH_2)_4$-$CH=CHCH_2CH=CH$-$(CH_2)_7$-$CO$-].

A-30912 factor A has the structure of formula I wherein $R^1$, $R^3$ and $R^4$ are all OH and $R^2$ is H.

A-30912 factor B has the structure of formula I wherein $R^1$ and $R^2$ are both H and $R^3$ and $R^4$ are both OH.

A-30912 factor D has the structure of formula I wherein $R^1$, $R^2$, $R^3$ and $R^4$ are all H.

A-30912 factor H has the structure of formula I wherein $R^1$ and $R^4$ are both OH, $R^2$ is H and $R^3$ is $CH_3O$.

Antibiotic S 31794/F-1 is an antifungal cyclic peptide of formula I wherein R is myristoyl and $R^1$, $R^3$ and $R^4$ are OH and $R^2$ is $-CO-NH_2$.

Each factor is isolated from the A30912 complex which contains the other factors arbitrarily designated factors B, C, D, E, F, and G. The A-30912 complex and the individual factors A through G are disclosed by M. Hoehn and K. Michel in U.S. Patent No. 4,024,245. Antibiotic A-30912 factor A is identical to antibiotic A-22802 which is described by C. Higgins and K. Michel in U.S. Patent No. 4,024,246. Factor A has also been found to be identical to antibiotic echinocandin B [see F. Benz et al., Helv. Chim. Acta, 57, 2459 (1974) and Swiss Patent No. 568,386] and to antibiotic SL 7810/F [see C. Keller-Juslen et al. Tetrahedron Letters, 4147 (1976) and Belgium Patent No. 834,289].

Antibiotic A-30912 factor A is prepared by submerged aerobic fermentation using one of several different organisms, namely: (a) *Aspergillus rugulosus* NRRL 8113; (b) *Aspergillus nidulans* NRRL 8112; (c) *Aspergillus nidulans* var. *echinulatus* A-32204, NRRL 3860; (d) *Aspergillus rugulosus* NRRL 8039; or (e) *Aspergillus nidulans* var. *roseus* NRRL 11440.

Factor B has also been found to be identical to antibiotic echinocandin C [see R. Traber et al., Helv. Chim. Acta, 62, 1252 (1979)] and to antibiotic SL 7810/F-II [see Belgium Patent No. 834,289].

Antibiotic A-30912 factor B is prepared by submerged aerobic fermentation using one of several different organisms, namely: (a) *Aspergillus rugulosus* NRRL 8113; (b) *Aspergillus nidulans* var. *echinulatus* A-32204, NRRL 3860; (c) *Aspergillus rugulosus* NRRL 8039; or (d) *Aspergillus nidulans* var. *roseus* NRRL 11440.

Factor D has also been found to be identical to antibiotic echinocandin D [see R. Traber et al., Helv. Chim. Acta, 62, 1252 (1979)] and to antibiotic SL 7810/F-III [see Belgium Patent No. 834,289].

Antibiotic A-30912 factor D is prepared by submerged aerobic fermentation using one of several different organisms, namely: (a) *Aspergillus rugulosus* NRRL 8113; (b) *Aspergillus nidulans* var. *echinulatus* A-32204, NRRL 3860,; (c) *Aspergillus rugulosus* NRRL 8039 (see Belgian Patent No. 834,289); or (d) *Aspergillus nidulans* var. *roseus* NRRL 11440.

Factor H is a later-discovered antibiotic A-30912 factor, and it is disclosed in the copending application No. 80301913.2 entitled "ANTIBIOTIC A-30912 FACTOR H,"

Antibiotic A-30912 factor H is prepared by fermentation using one of several different organisms, namely: (a) Aspergillus rugulosus NRRL 8113; or (b) Aspergillus nidulans var. roseus NRRL 11440.

A subculture of A. nidulans var. roseus has been deposited and made a part of the permanent culture collection of the Northern Regional Research Laboratory, U.S. Department of Agriculture, Agricultural Research Service, Peoria, Illinois 61604, from which it is available to the public under the number NRRL 11440.

When a strain of A nidulans var. roseus NRRL 11440 is used to produce any one of the A-30912 factors a complex of factors is obtained which for convenience is called the A-42355 antibiotic complex. A-30912 factor A is the major factor of the A-42355 antibiotic complex, while factors B, D and H are minor factors. Preparations 2 to 7 herein, illustrate the preparation of the A-42355 complex and the isolation and purification of the individual A-30912 factors therefrom.

In the antibiotic molecule of formula I, the linoleoyl side chain (R) is attached at the cyclic peptide nucleus at the α-amino group of the ornithine residue. Surprisingly, it has been found that the linoleoyl side chain can be cleaved from the nucleus by an enzyme without affecting the chemical intregity of the nucleus. The enzyme employed to effect the deacylation reaction is produced by a microorganism of the family Actinoplanaceae, preferably the microorganism Actinoplanes utahensis NRRL 12052, or a variant thereof. To accomplish deacylation, the appropriate antibiotic A30912 factor is

added to a culture of the microorganism and the culture is allowed to incubate with the substrate until the deacylation is subtantially complete. The cyclic nucleus thereby obtained is separated from the fermentation broth by methods known in the art. Unlike the antibiotic A-30912 factors A, B, D and H, the cyclic nucleus (lacking the linoleoyl side chain) is substantially devoid of antifungal activity.

Antibiotic S31794/F-1, which is disclosed in German Offenlegungschrift 2,628,965 and U.S. Patent No. 4,173,629, is produced by *Acrophialophora limonispora nov. spec.* Dreyfuss et Muller NRRL 8095. S31794/F-1 has the following characteristics: m.p. 178-180°C. (dec.) (amorphous) or 181-183°C. (dec.) (crystalline); $[\alpha]_D^{20}$ -24° (*c* 0.5, $CH_3OH$) or +37° (*c* 0.5, pyridine) (crystalline); UV absorption maxima in methanol at 194 nm ($E_{1cm}^{1\%}$ = 807), 225 nm (shoulder) $E_{1cm}^{1\%}$ = 132), 276 nm ($E_{1cm}^{1\%}$ = 12.8), 284 nm (shoulder) $E_{1cm}^{1\%}$ = 10.5); $^{13}C$-NMR spectrum in deuteromethanol (190 mg in 1.5 ml deuteromethanol, tetramethylsilane as internal standard) with the following characteristics (crystalline):

| PPM | PPM | PPM |
|---|---|---|
| 176.2 | 75.5 | 51.2 |
| 175.0 | 74.0 | 39.7 |
| 173.7 | 71.0 | 38.8 |
| 172.6 | 70.5 | 36.6 |
| 172.0 | 69.7 | 34.8 |
| 171.8 | 68.0 | 32.8 |
| 171.7 | 62.2 | 30.6 |
| 168.6 | 58.3 | 26.7 |
| 157.7 | 57.0 | 23.5 |
| 132.5 | 56.2 | 19.7 |
| 129.0 | 55.4 | 14.3 |
| 115.9 | 52.9 | 11.1 |
| 76.6 | | |

an approximate elemental analysis (after drying crystalline material for two hours in a high vacuum at 100°C) as follows: 55.5-56.5 percent carbon, 7.5-7.7 percent hydrogen, 10.5-10.8 percent nitrogen and 25.5-26.0 percent oxygen; is readily soluble in methanol, ethanol, pyridine, dimethyl sulfoxide and poorly soluble in water, chloroform, ethyl acetate, diethyl ether, benzene and hexane; and has antifungal activity, especially against *Candida albicans*.

Antibiotic S31794/F-1 is prepared by submerged aerobic cultivation of *Acrophialophora limonispora* NRRL 8095 as described in Preparations 8 and 9. This microorganism is a part of the permanent culture collection of the Northern Regional Research Center, U.S. Department of Agriculture, Agricultural Research Culture Collection, North Central Region, Peoria, Illinois 61604, from which it is available to the public under the designated NRRL number.

Antibiotic S31794/F-1 has antifungal activity, particularly against *Candida* strains such as *Candida albicans*. Thus, production and isolation of the antibiotic can be monitored by bioautography using a *Candida* species such as *Candida albicans*.

In the antibiotic S31794/F-1 molecule of formula I, wherein $R^1$, $R^3$ and $R^4$ are all OH, and $R^2$ is $-CO-NH_2$, the myristoyl side chain (R) is attached at the cyclic peptide nucleus at the α-amino group of the dihydroxyornithine residue. Surprisingly, it has been found that the myristoyl side chain can be cleaved from the nucleus by an enzyme without affecting the chemical integrity of the nucleus. The enzyme employed to effect the deacylation reaction is produced by a microorganism of the family *Actinoplanaceae*, preferably the microorganism *Actinoplanes utahensis* NRRL 12052, or a variant thereof. To accomplish deacylation, antibiotic S31794/F-1 is added to a culture of the microorganism and the culture is allowed to incubate with the substrate until the deacylation is subtantially complete. The cyclic nucleus thereby obtained is separated from the fermentation broth by methods known in the art. Unlike antibiotic S31794/F-1, the cyclic nucleus (lacking the myristoyl side chain) is substantially devoid of antifungal activity.

The cyclic peptide nuclei afforded by the aforedescribed enzymatic deacylations of the antibiotics of formula I are depicted in general formula II.

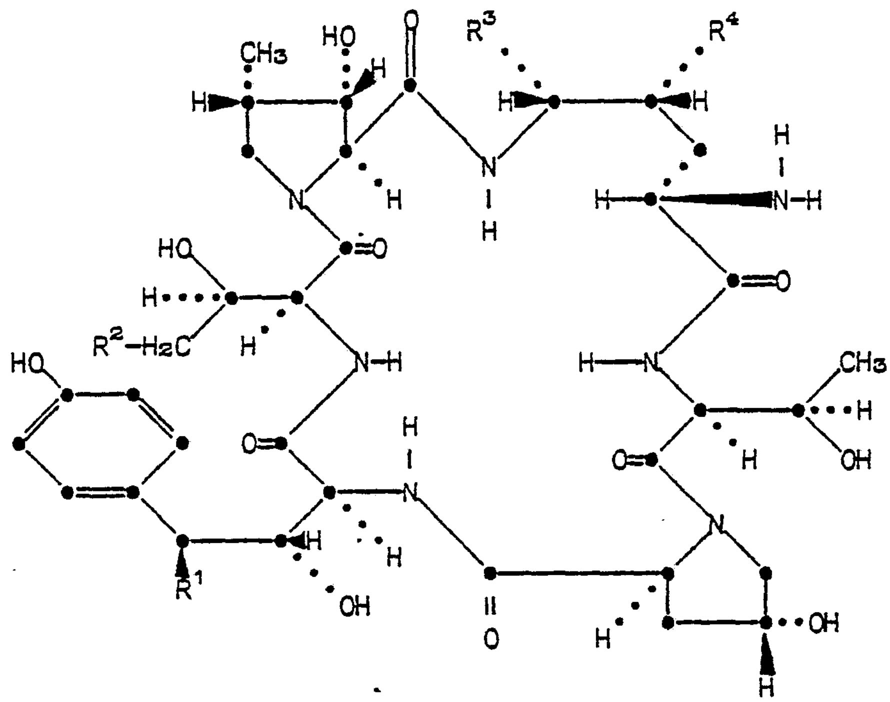

II

The compound of formula II wherein $R^1$, $R^3$ and $R^4$ are all OH and $R^2$ is H is the A-30912 factor A nucleus and for convenience will be referred to herein as the "A-30912A nucleus". A-30912A nucleus has an empirical formula of $C_{34}H_{51}N_7O_{15}$ and a molecular weight of 797.83.

The compound of formula II wherein $R^1$ and $R^2$ are both H and $R^3$ and $R^4$ are both OH is the A-30912 factor B nucleus and for convenience will be referred to herein as the "A-30912 B nucleus". A-30912 B nucleus has an empirical formula of $C_{34}H_{51}N_7O_{14}$ and a molecular weight of 781.81.

The compound of formula II wherein $R^1$, $R^2$, $R^3$ and $R^4$ are all H is the A-30912 factor D nucleus and for convenience will be referred to herein as the "A-30912D nucleus". A-30912D nucleus has an empirical formula of $C_{34}H_{51}N_7O_{12}$ and a molecular weight of 749.83.

The compound of formula II wherein $R^1$ and $R^4$ are both OH, $R^2$ is H and $R^3$ is $CH_3O$- is the A-30912 factor H nucleus and for convenience, will be referred to herein as the "A-30912H nucleus".

The compound of formula II wherein $R^1$, $R^3$ and $R^4$ are all OH and $R^2$ is $-CO-NH_2$ is the nucleus of the S 31794/F-1 antibiotic and will be referred to herein as the "S 31794/F-1 nucleus". The S 31794/F-1 nucleus has an empirical formula of $C_{35}H_{52}N_8O_{16}$ and a molecular weight of 840.87.

Removal of the side chain group affords a free primary α-amino group in the ornithine residue of the cyclic peptide. As will be apparent to those skilled in the art, the nuclei can be obtained either in the form of the free amine or of the acid addition salt. Although any suitable acid addition salt may be employed, those which are non-toxic and pharmaceutically acceptable are preferred.

The method of preparing each nucleus from the appropriate antibiotic by means of fermentation using Actinoplanes utahensis NRRL 12052 is described in the co-pending application of Bernard J. Abbott and David S. Fukuda, entitled "CYCLIC PEPTIDE NUCLEI", Docket No. X-5399A, which is being filed herewith this even date.

Cultures of representative species of *Actinoplanaceae* are available to the public from the Northern Regional Research Laboratory under the following accession numbers:

| | |
|---|---|
| *Actinoplanes utahensis* | NRRL 12052 |
| *Actinoplanes missouriensis* | NRRL 12053 |
| *Actinoplanes sp.* | NRRL 8122 |
| *Actinoplanes sp.* | NRRL 12065 |
| *Streptosporangium roseum* var. *hollandensis* | NRRL 12064 |

The effectiveness of any given strain of microorganism within the family *Actinoplanaceae* for carrying out the deacylation of this invention is determined by the following procedure. A suitable growth medium is inoculated with the microorganism. The culture is incubated at about 28°C. for two or three days on a rotary shaker. One of the substrate antibiotics is then added to the culture. The pH of the fermentation medium is maintained at about pH 6.5. The culture is monitored for activity using a *Candida albicans* assay. Loss of antibiotic activity is an indication that the microorganism produces the requisite enzyme for deacylation. This must be verified, however, using one of the following methods: 1) analysis by HPLC for presence of the intact nucleus; or 2) reacylation with an appropriate side chain (e.g. linoleoyl, stearoyl, palmitoyl or myristoyl) to restore activity.

It is known that other antibiotic substances possess the same nucleus as that of antibiotic A-30912 factor A. These antibiotics differ from antibiotic

A-30912 factor A in that different acyl groups are present in place of the linoleoyl group (R) in Formula I. Such antibiotics are: (a) tetrahydro-A-30912 factor A (tetrahydro-SL 7810/F; tetrahydro echinocandin B) described in Belgium Patent 834,289 and by F. Benz et al., Helv. Chim. Acta, 57 2459 (1974), which compound is depicted in Formula I when R is stearoyl; and (b) aculaecin A, which is a component of the aculaecin complex (prepared by fermentation using *Aspergillus aculeatus* NRRL 8075) and is described by K. Mizuno et al., in U.S. Patent 3,978,210. As is discussed in Belgium Patent 859,067, in aculaecin A the palmitoyl side chain is present in place of linoleoyl. Tetrahydro-A-30912 factor A can be prepared from antibiotic A-30912 factor A by catalytic hydrogenation using $PtO_2$ in ethanol under positive pressure. Both tetrahydro-A-30912 factor A and aculaecin A can be employed as substrates for the enzymatic deacylation using the procedures herein described.

It is also known that another antibiotic substance possesses the same nucleus as that of antibiotic A-30912 factor B. This substance, which differs from antibiotic A-30912 factor B in that a different acyl group is present in place of the linoleoyl group (R) in Formula I, is tetrahydro-A-30912 factor B (tetrahydro-SL 7810/F-II; tetrahydro echinocandin C) described by R. Traber et al., Helv. Chim. Acta, 62 1252 (1979). Tetrahydro-A-30912 factor B is depicted in Formula I when R is stearoyl. Tetrahydro-A-30912 factor B can be prepared from antibiotic A-30912 factor

B by catalytic hydrogenation using $PtO_2$ in ethanol under positive pressure. Tetrahydro-A-30912 factor B can be employed as a substrate in place of antibiotic A-30912 factor B for the enzymatic deacylation using the procedures herein described.

Additionally, it is known that another antibiotic substance possesses the same nucleus as that of antibiotic A-30912 factor D. This substance, which differs from antibiotic A-30912 factor D in that a different acyl group is present in place of the linoleoyl group (R) in Formula I, is tetrahydro-A-30912 factor D (tetrahydro-SL 7810/F-III; tetrahydro echinocandin D) described by R. Traber et al., Helv. Chim. Acta, 62 1252 (1979). Tetrahydro-A-30912 factor B is depicted in Formula I when R is stearoyl. Tetrahydro-A-30912 factor D can be prepared from antibiotic A-30912 factor D by catalytic hydrogenation using $PtO_2$ in ethanol under positive pressure. Tetrahydro-A-30912 factor D can be employed as a substrate in place of antibiotic A-30912 factor D for the enzymatic deacylation using the procedures herein described.

In antibiotic A-30912 factor H, the 5-hydroxyl group present in the dihydroxy ornithine residue of the peptide nucleus is methylated, while in antibiotic A-30912 factor A, the 5-hydroxyl group is unsubstituted. It will be recognized, therefore, that factor H can be made synthetically by methylating factor A using methods that are conventional for preparing an aliphatic ether from an alcohol. It will also be recognized that Factor A can be alkylated with

other lower alkyl groups to form alkyloxy homologs of the factor H molecule. The alkyloxy homologs of Factor H, which can be prepared synthetically from factor A, are known as the A-30912 factor H-type homologs. The compound of formula II wherein $R^1$ and $R^4$ are both OH, $R^2$ is H and $R^3$ is $C_2$-$C_6$ alkyloxy are herein referred to as the "A-30912H-type nuclei".

It will also be apparent that the linoleoyl side chain of the A-30912 factor H or of the A-30912 factor H-type homologs can be hydrogenated using conventional techniques to provide tetrahydro-A-30912 factor H or the corresponding tetrahydro derivative of the alkyloxy homologs (R is stearoyl). Alternatively, the tetrahydro derivatives can be made by first hydrogenating antibiotic A-30912 factor A to give tetrahydro-A-30912 factor A and then forming the desired alkyloxy derivative therefrom.

It will be understood that antibiotic A-30912 factor H, tetrahydro-A-30912 factor H, a $C_2$-$C_6$ alkyloxy homolog of factor H, or a tetrahydro derivative of a $C_2$-$C_6$ alkyloxy homolog of factor H can be employed as a substrate for the enzymatic deacylation using the procedures herein described.

The invention sought to be patented comprehends novel compounds derived by acylating a cyclic peptide nuclei of formula II. The compounds of the present invention have the chemical structure depicted in formula III:

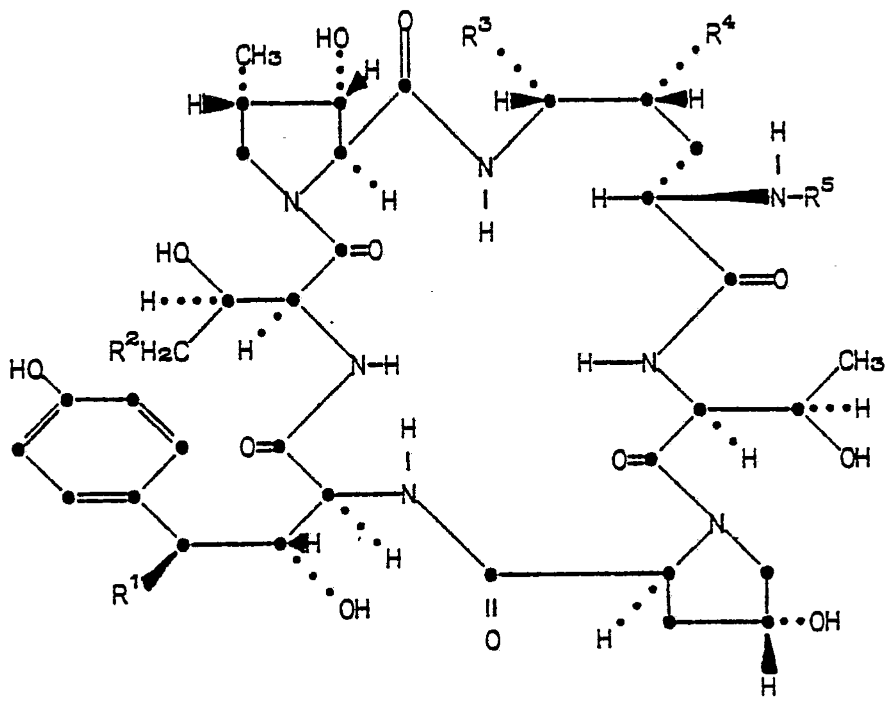

III

wherein $R^1$ is H or OH and;

when $R^1$ is H, $R^2$ is H and $R^3$ and $R^4$ are both H or both OH,

and

when $R^1$ is OH, $R^2$ is H, $R^3$ is OH or $C_1$-$C_6$ alkyloxy and $R^4$ is OH, or $R^2$ is -CO-$NH_2$ and $R^3$ and $R^4$ are both OH;

$R^5$ is an N-alkanoyl amino acyl group of the formula $-W-\overset{O}{\overset{\|}{C}}-R^6$ wherein:

W is a divalent aminoacyl radical of the formula:

(a) $-\overset{O}{\overset{\|}{C}}-A-NH-$

wherein A is $C_1$-$C_{10}$ alkylene or $C_5$-$C_6$ cycloalkylene;

(b) $-\overset{O}{\overset{\|}{C}}-\overset{R^7}{\overset{|}{C}}H-NH-$

wherein $R^7$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$-$C_3$ alkyl, hydroxy, $C_1$-$C_3$ alkylthio, carbamyl, or $C_1$-$C_3$ alkylcarbamyl;

(c)

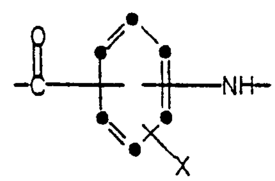

wherein X is hydrogen chloro, bromo, iodo, nitro, $C_1$-$C_3$ alkyl, hydroxy, $C_1$-$C_3$ alkoxy, mercapto, $C_1$-$C_3$ alkylthio, carbamyl, or $C_1$-$C_3$ alkylcarbamyl;

(d)

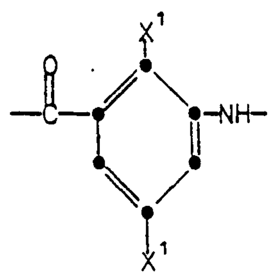

or

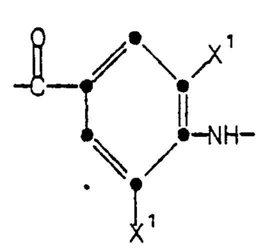

wherein $X^1$ is chloro, bromo, or iodo;

(e) 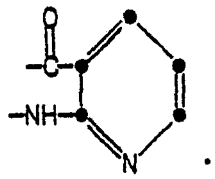 or 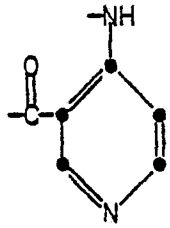 ; or

(f) 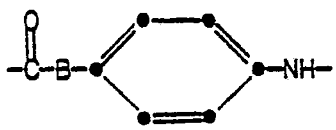

wherein B is a divalent radical of the formula: $-(CH_2)_n-$, wherein n is an integer from 1 to 3; $-CH=CH-$; $-CH=CH-CH_2-$; or $-\overset{O}{\overset{\|}{C}}NHCH_2-$

and $R^6$ is $C_1$-$C_{17}$ alkyl or $C_2$-$C_{17}$ alkenyl.

As employed herein the terms "alkylene", "alkyl", "alkoxy", "alkylthio", and "alkenyl" comprehend both straight and branched hydrocarbon chains. "Alkyl" means a univalent saturated hydrocarbon radical. "Alkenyl" means a univalent unsaturated hydrocarbon radical containing one, two, or three double bonds, which may be oriented in the <u>cis</u> or <u>trans</u> configuration. "Alkylene" means a divalent saturated hydrocarbon radical. "Cycloalkylene" means a divalent cyclic saturated hydrocarbon radical.

Illustrative $C_1$-$C_{10}$ alkylene radicals, which are preferred for purposes of this invention are:

$-CH_2-$; $\overset{R^8}{-CH-}$ in which $R^8$ is $C_1$-$C_4$ alkyl (i.e., methyl, ethyl, n-propyl, i-propyl, n-butyl, t-butyl, i-butyl, or 1-methylpropyl); $-(CH_2)_m-$ in which m is an integer from 2 to 10; and $CH_3-(CH_2)_q-CH-(CH_2)_p-$, in which p is an integer from 1 to 8 and q is an integer from 0 to 7, provided that n + m must be no greater than 8.

Illustrative $C_1$-$C_{17}$ alkyl groups which are preferred for the purposes of this invention are:

(a) $CH_3-$;

(b) $-(CH_2)_nCH_3$ wherein n is an integer from 1 to 16; and

(c) $-(CH_2)_r\overset{CH_3}{CH}(CH_2)_sCH_3$ wherein r and s are independently, an integer from 0 to 14 provided that r + s can be no greater than 14.

Illustrative $C_2$-$C_{17}$ alkenyl radicals, which are preferred for the purpose of this invention, are

(a) $-(CH_2)_t-CH=CH-(CH_2)_u-CH_3$ wherein t and u are independently, an integer from 0 to 14 provided that t + u can be no greater than 14.

(b) $-(CH_2)_v-CH=CH-(CH_2)_y-CH=CH-(CH_2)_z-CH_3$ wherein v and z are independently, an integer from 0 to 11 and y is an integer from 1 to 12 provided that v + y + z can be no greater than 11.

In particular, the following embodiments of the $C_1$-$C_{17}$ alkyl groups are preferred:

$CH_3$-

$CH_3(CH_2)_5$-

$CH_3(CH_2)_6$-

$CH_3(CH_2)_8$-

$CH_3(CH_2)_{10}$-

$CH_3(CH_2)_{12}$-

$CH_3(CH_2)_{14}$-

$CH_3(CH_2)_{16}$-

In particular, the following embodiments of the $C_2$-$C_{17}$ alkenyl groups are preferred:

cis-$CH_3(CH_2)_5CH{=}CH(CH_2)_7$-

trans-$CH_3(CH_2)_5CH{=}CH(CH_2)_7$-

cis-$CH_3(CH_2)_{10}CH{=}CH(CH_2)_4$-

trans-$CH_3(CH_2)_{10}CH{=}CH(CH_2)_4$-

cis-$CH_3(CH_2)_7CH{=}CH(CH_2)_7$-

trans-$CH_3(CH_2)_7CH{=}CH(CH_2)_7$-

cis-$CH_3(CH_2)_5CH{=}CH(CH_2)_9$-

trans-$CH_3(CH_2)_5CH{=}CH(CH_2)_9$-

cis, cis-$CH_3(CH_2)_4CH{=}CHCH_2CH{=}CH(CH_2)_7$-

trans, trans-$CH_3(CH_2)_4CH{=}CHCH_2CH{=}CH(CH_2)_7$-

cis,cis,cis-$CH_3CH_2CH{=}CHCH_2CH{=}CHCH_2CH{=}CH$-$(CH_2)_7$-.

When "W" is a divalent radical of the formula

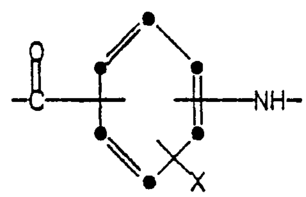

it will be recognized by those skilled in the art that the $-\overset{O}{\overset{\|}{C}}-$ function and the -NH- function may be oriented on the benzene ring in the *ortho*, *meta*, or *para* configuration relative to each other. The substituent represented by X may be substituted at any available position of the benzene ring. Preferred embodiments are those in which X is hydrogen and the $-\overset{O}{\overset{\|}{C}}-$ and -NH- functions are oriented in the *para* configuration.

The terms "substituted phenyl" and "substituted benzyl", as defined by $R^7$ in Formula III, contemplate substitution of a group at any of the available positions in the benzene ring--i.e. the substituent may be in the *ortho*, *meta*, or *para* configuration. The term "$C_1$-$C_3$ alkyl" as defined by $R^7$ or X in Formula III includes the methyl, ethyl, *n*-propyl, or *i*-propyl groups.

Specifically, the invention provides a compound of Formula:

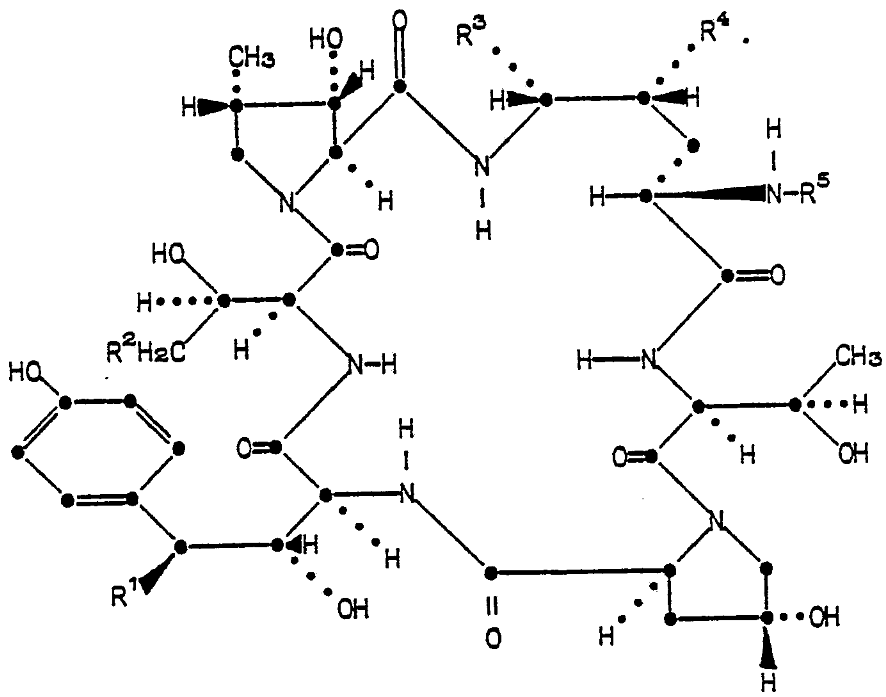

III

wherein $R^1$ is H or OH and;

when $R^1$ is H, $R^2$ is H and $R^3$ and $R^4$ are both H or both OH,

and

when $R^1$ is OH, $R^2$ is H, $R^3$ is OH or $C_1$-$C_6$ alkyloxy and $R^4$ is OH, or $R^2$ is $-CO-NH_2$ and $R^3$ and $R^4$ are both OH;

$R^5$ is an N-alkanoyl amino acyl group of the formula $-W-\overset{O}{\overset{\|}{C}}-R^6$ wherein:

W is a divalent aminoacyl radical of the formula:

(a) $-\overset{O}{\overset{\|}{C}}-A-NH-$

wherein A is $C_1$-$C_{10}$ alkylene or $C_5$-$C_6$ cycloalkylene;

(b) $-\overset{O}{\overset{\|}{C}}-\overset{R^7}{\overset{|}{C}H}-NH-$

wherein $R^7$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$-$C_3$ alkyl, hydroxy, $C_1$-$C_3$ alkylthio, carbamyl, or $C_1$-$C_3$ alkylcarbamyl;

(c) 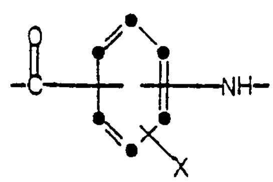

wherein X is hydrogen chloro, bromo, iodo, nitro, $C_1$-$C_3$ alkyl, hydroxy, $C_1$-$C_3$ alkoxy, mercapto, $C_1$-$C_3$ alkylthio, carbamyl, or $C_1$-$C_3$ alkylcarbamyl;

(d) 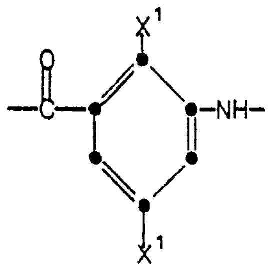 or 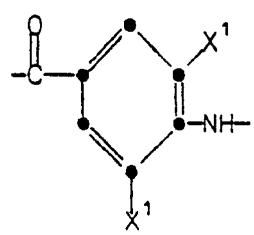

wherein $X^1$ is chloro, bromo, or iodo;

(e) 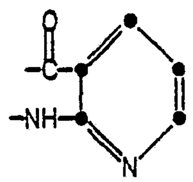 or 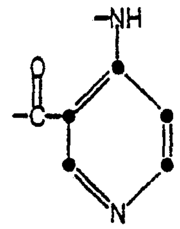 ; or

(f) 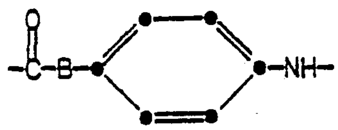

wherein B is a divalent radical of the formula: $-(CH_2)_n-$, wherein n is an integer from 1 to 3; $-CH=CH-$; $-CH=CH-CH_2-$; or

$-\overset{O}{\overset{\|}{C}}NHCH_2-$

and $R^6$ is $C_1$-$C_{17}$ alkyl or $C_2$-$C_{17}$ alkenyl.

The compounds of Formula III inhibit the growth of pathogenic fungi as evidenced by standard biological test procedures. The compounds are useful, therefore, for controlling the growth of fungi on environmental surfaces (as an antiseptic) or in treating infections caused by fungi. The antifungal activity of the compounds has been demonstrated against <u>Candida albicans</u> <u>in vitro</u> in agar plate disc diffusion tests and in agar dilution tests, or <u>in vivo</u> in tests in mice infected with <u>C. albicans</u>. Thus, the compounds are particularly useful in treating infections caused by strains of <u>C. albicans</u> (candidosis). The compounds of Formula III have also shown activity <u>in vitro</u> in

agar-plate disc diffusion tests against Trichophyton mentagrophytes, a dermatophytic organism. Activity has also been found in in vitro agar plate disc diffusion tests against Saccharomyces pastorianus, and Neurospora crassa. Certain compounds (as shown in Example 40, Table 8) give significant blood levels upon oral administration in mice.

When given to a dog by intravenous administration, 100 mg/kg per day for five days, the compound of Formula III wherein $R^1$, $R^3$ and $R^4$ are all OH, $R^2$ is H and $R^5$ is n-dodecanoyl-p-aminobenzoyl showed no outward signs of toxicity, although increased SGPT levels were observed.

The compounds of Formula III are prepared by acylating the appropriate nucleus at the α-amino group of ornithine with the appropriate N-alkanoyl aminoacyl or N-alkenoyl amino acyl side chain using methods conventional in the art for forming an amide bond. The acylation is accomplished, in general, by reacting the nucleus with an $R^5$ introducing acylating agent, for example an activated derivative of the acid (Formula IV) corresponding to the desired acyl side chain group.

$$HO-W-\overset{\overset{O}{\|}}{C}-R^6$$

IV

(W and $R^6$ have the meaning described herein supra).

By the term "activated derivative" is meant a derivative which renders the carboxyl function of the acylating agent reactive to coupling with the primary amino group to form the amide bond which links the acyl side chain

to the nucleus. Suitable activated derivatives, their methods of preparation, and their methods of use as acylating agents for a primary amine will be recognized by those skilled in the art. Preferred activated derivatives are: (a) an acid halide (e.g. acid chloride), (b) an acid anhydride (e.g. an alkoxyformic acid anhydride or aryloxyformic acid anhydride) or (c) an activated ester (e.g. a 2,4,5-trichlorophenyl ester, a N-hydroxybenztriazole ester, or an N-hydroxysuccinimide ester). Other methods for activating the carboxyl function include reaction of the carboxylic acid with a carbonyldiimide (e.g. N,N-dicyclohexylcarbodiimide or N,N'-diisopropylcarbodiimide) to give a reactive intermediate which, because of instability, is not isolated, the reaction with the primary amine being carried out *in situ*.

A preferred method for preparing the compounds of Formula III is by the active ester method. The use of the 2,4,5-trichlorophenyl ester of the desired N-alkanoylamino acid or N-alkenoylamino acid (Formula IV) as the acylating agent is most preferred. In this method, an excess amount of the active ester is reacted with the nucleus at room temperature in a nonreactive organic solvent such as dimethyl formamide (DMF). The reaction time is not critical, although a time of about 15 to about 18 hours is preferred. At the conclusion of the reaction, the solvent is removed, and the residue is purified such as by column chromatography using silica gel as the stationary phase and a mixture of ethyl acetate/methanol (3:2, v/v) as the solvent system.

The 2,4,5-trichlorophenyl esters of the N-alkanoylamino acids or N-alkenoylamino acids can be prepared conveniently by treating the desired amino acid (Formula IV) with 2,4,5-trichlorophenol in the presence of a coupling agent, such as N,N'-dicyclohexylcarbodiimide. Other methods suitable for preparing amino acid esters will be apparent to those skilled in the art.

The N-alkanoylamino acids or N-alkenoylamino acids are either known compounds or they can be made by acylating the appropriate amino acid with the appropriate alkanoyl or alkenoyl group using conventional methods, such as those described herein supra. A preferred way of preparing the N-alkanoylamino acids is by treating the appropriate amino acid with an alkanoic acid chloride in pyridine. The alkanoic acids or alkenoic acids, the activated derivatives thereof, and the amino acids employed in the preparation of the products of this invention are either known compounds or they can be made by known methods or by modification of known methods which will be apparent to those skilled in the art.

If a particular amino acid contains an acylable functional group other than the amino group, it will be understood by those skilled in the art that such a group must be protected prior to reaction of the amino acid with the reagent employed to attach the alkanoyl or alkenoyl group. Suitable protecting groups can be any group known in the art to be useful for the protection of a side chain functional group in peptide

synthesis. Such groups are well known, and the selection of a particular protecting group and its method of use will be readily known to one skilled in the art [see, for example, "Protective Groups In Organic Chemistry", M. McOmie, Editor, Plenum Press, N.Y., 1973].

It will be recognized that certain amino acids employed in the synthesis of the products of this invention may exist in optically active forms, and both the natural configuration (L-configuration) and unnatural configuration (D-configuration) may be employed as starting materials and will give products which are within the contemplation of this invention.

When employed systemically, the dosage of the compounds of Formula III will vary according to the particular compound being employed, the severity and nature of the infection, and the physical condition of the subject being treated. Therapy should be initiated at low dosages, the dosage being increased until the desired antifungal effect is obtained. The compounds can be administered intravenously or intramuscularly by injection in the form of a sterile aqueous solution or suspension to which may be added, if desired, various conventional pharmaceutically acceptable preserving, buffering, solubilizing, or suspending agents. Other additives, such as saline or glucose may be added to make the solutions isotonic. The proportions and nature of such additives will be apparent to those skilled in the art.

Certain compounds of Formula III give significant blood levels after oral administration (see

Example 40, Table 8) and can be administered systemically by the oral route. For oral use, such compounds can be administered in combination with pharmaceutically acceptable carriers or excipients in the form of capsules, tablets or powders. The nature and proportion of such carriers or excipients of which will be recognized by those skilled in the art.

When employed to treat vaginal candida infections, the compounds of Formula III can be administered in combination with pharmaceutically acceptable conventional excipients suitable for intravaginal use. Formulations adapted for intravaginal administration will be known to those skilled in the art.

The methods of making and using the compounds of the present invention are illustrated in the following examples:

Preparation 1

Fermentation of Actinoplanes utahensis NRRL 12052

A stock culture of Actinoplanes utahensis NRRL 12052 is prepared and maintained on an agar slant. The medium used to prepare the slant is selected from one of the following:

MEDIUM A

| Ingredient | Amount |
|---|---|
| Baby oatmeal | 60.0 g |
| Yeast | 2.5 g |
| $K_2HPO_4$ | 1.0 g |
| Czapek's mineral stock* | 5.0 ml |
| Agar | 25.0 g |
| Deionized water | q.s. to 1 liter |

pH before autoclaving is about 5.9; adjust to pH 7.2 by addition of NaOH; after autoclaving, pH is about 6.7.

*Czapek's mineral stock has the following composition:

| Ingredient | Amount |
|---|---|
| $FeSO_4 \cdot 7H_2O$ (dissolved in 2 ml conc HCl) | 2 g |
| KCl | 100 g |
| $MgSO_4 \cdot 7H_2O$ | 100 g |
| Deionized water | q.s. to 1 liter |

MEDIUM B

| Ingredient | Amount |
|---|---|
| Potato dextrin | 5.0 g |
| Yeast extract | 0.5 g |
| Enzymatic hydrolysate of casein* | 3.0 g |
| Beef extract | 0.5 g |
| Dextrose | 12.5 g |
| Corn starch | 5.0 g |
| Meat peptone | 5.0 g |
| Blackstrap molasses | 2.5 g |
| $MgSO_4 \cdot 7H_2O$ | 0.25 g |
| $CaCO_3$ | 1.0 g |
| Czapek's mineral stock | 2.0 ml |
| Agar | 20.0 g |
| Deionized water | q.s. to 1 liter |

*N-Z-Amine A, Humko Sheffield Chemical, Lyndhurst, N.J.

The slant is inoculated with <u>Actinoplanes utahensis</u> NRRL 12052, and the inoculated slant is incubated at 30°C for about 8 to 10 days. About 1/2 of the slant growth is used to inoculate 50 ml of a vegetative medium having the following composition:

| Ingredient | Amount |
|---|---|
| Baby oatmeal | 20.0 g |
| Sucrose | 20.0 g |
| Yeast | 2.5 g |
| Distiller's Dried Grain* | 5.0 g |
| $K_2HPO_4$ | 1.0 g |
| Czapek's mineral stock | 5.0 ml |
| Deionized water | q.s. to 1 liter |

adjust to pH 7.4 with NaOH; after autoclaving, pH is about 6.8.

*National Distillers Products Co., 99 Park Ave., New York, N.Y.

The inoculated vegetative medium is incubated in a 250-ml wide-mouth Erlenmeyer flask at 30°C for about 72 hours on a shaker rotating through an arc two inches in diameter at 250 RPM.

This incubated vegetative medium may be used directly to inoculate a second-stage vegetative medium. Alternatively and preferably, it can be stored for later use by maintaining the culture in the vapor phase of liquid nitrogen. The culture is prepared for such storage in multiple small vials as follows: In each vial is placed 2 ml of incubated vegetative medium and 2 ml of a glycerol-lactose solution [see W. A.

Dailey and C. E. Higgens, "Preservation and Storage of Microorganisms in the Gas Phase of Liquid Nitrogen, Cryobiol 10, 364-367 (1973) for details]. The prepared suspensions are stored in the vapor phase of liquid nitrogen.

A stored suspension (1 ml) thus prepared is used to inoculate 50 ml of a first-stage vegetative medium (having the composition earlier described). The inoculated first-stage vegetative medium is incubated as above-described.

In order to provide a larger volume of inoculum, 10 ml of the incubated first-stage vegetative medium is used to inoculate 400 ml of a second-stage vegetative medium having the same composition as the first-stage vegetative medium. The second-stage medium is incubated in a two-liter wide-mouth Erlenmeyer flask at 30°C for about 48 hours on a shaker rotating through an arc two inches in diameter at 250 RPM.

Incubated second-stage vegetative medium (800 ml), prepared as above-described, is used to inoculate 100 liters of sterile production medium selected from one of the following:

MEDIUM I

| Ingredient | Amount (g/L) |
|---|---|
| Peanut meal | 10.0 |
| Soluble meat peptone | 5.0 |
| Sucrose | 20.0 |
| $KH_2PO_4$ | 0.5 |
| $K_2HPO_4$ | 1.2 |
| $MgSO_4 \cdot 7H_2O$ | 0.25 |
| Tap water | q.s. to 1 liter |

The pH of the medium is about 6.9 after sterilization by autoclaving at 121°C for 45 minutes at about 16-18 psi.

MEDIUM II

| Ingredient | Amount (g/L) |
|---|---|
| Sucrose | 30.0 |
| Peptone | 5.0 |
| $K_2HPO_4$ | 1.0 |
| KCl | 0.5 |
| $MgSO_4 \cdot 7H_2O$ | 0.5 |
| $FeSO_4 \cdot 7H_2O$ | 0.002 |
| Deionized water | q.s. to 1 liter |

Adjust to pH 7.0 with HCl; after autoclaving, pH is about 7.0.

MEDIUM III

| Ingredient | Amount (g/L) |
|---|---|
| Glucose | 20.0 |
| $NH_4Cl$ | 3.0 |
| $Na_2SO_4$ | 2.0 |
| $ZnCl_2$ | 0.019 |
| $MgCl_2 \cdot 6H_2O$ | 0.304 |
| $FeCl_3 \cdot 6H_2O$ | 0.062 |
| $MnCl_2 \cdot 4H_2O$ | 0.035 |
| $CuCl_2 \cdot 2H_2O$ | 0.005 |
| $CaCO_3$ | 6.0 |
| $KH_2PO_4$* | 0.67 |
| Tap water | q.s. to 1 liter |

*Sterilized separately and added aseptically
Final pH about 6.6.

The inoculated production medium is allowed to ferment in a 165-liter fermentation tank at a temperature of about 30°C for about 42 hours. The fermentation medium is stirred with conventional agitators at about 200 RPM and aerated with sterile air to maintain the dissolved oxygen level above 30% of air saturation at atmospheric pressure.

## Preparation 2

## Preparation of the A-42355 Antibiotic Complex

### A. Shake-Flask Fermentation

A culture of *Aspergillus nidulans* var. *roseus* NRRL 11440 is prepared and maintained on an agar slant prepared with medium having the following composition:

| Ingredient | Amount |
|---|---|
| Glucose | 5 g |
| Yeast extract | 2 g |
| $CaCO_3$ | 3 g |
| Vegetable juice* | 200 ml |
| Agar** | 20 g |
| Deionized water | q.s. to 1 liter |

(initial pH 6.1)

*V-8 Juice, Campbell Soup Co., Camden, N.J.

**Meer Corp.

The slant is inoculated with *Aspergillus nidulans* var. *roseus* NRRL 11440, and the inoculated slant is incubated at 25°C. for about seven days. The mature slant culture is covered with water and scraped with a sterile loop to loosen the spores. The resulting suspension is further suspended in 10 ml of sterile deionized water.

One ml of the suspended slant growth is used to inoculate 55 ml of vegetative medium in a 250-ml flask. The vegetative medium has the following composition:

| Ingredient | Amount |
|---|---|
| Sucrose | 25 g |
| Blackstrap molasses | 36 g |
| Corn-steep liquor | 6 g |
| Malt extract | 10 g |
| $K_2HPO_4$ | 2 g |
| Enzymatic hydrolysate of casein* | 10 g |
| Tap water | 1100 ml |

(initial pH 6.5-6.7)

*N-Z-Case, Humko Sheffield Chemical, Lyndhurst, N.J.

The inoculated vegetative medium is incubated at 25°C. for 48 hours at 250 rpm on a rotary-type shaker. After 24 hours, the medium is homogenized for one minute at low speed in a blender (Waring type) and then returned to incubation for the remaining 24 hours. Alternatively, the inoculated vegetative medium can be incubated for 48 hours and then homogenized for 15 seconds at low speed.

This incubated vegetative medium may be used to inoculate shake-flask fermentation culture medium or to inoculate a second-stage vegetative medium. Alternatively, it can be stored for later use by maintaining the culture in the vapor phase of liquid nitrogen. The culture is prepared for such storage in multiple small vials as follows:

The vegetative cultures are mixed volume/volume with a suspending solution having the following composition:

| Ingredient | Amount |
| --- | --- |
| Glycerol | 20 ml |
| Lactose | 10 g |
| Deionized water | q.s. to 100 ml |

The prepared suspensions are distributed in small sterile screw-cap tubes (4 ml per tube). These tubes are stored in the vapor phase of liquid nitrogen.

A stored suspension thus prepared can be used to inoculate either agar slants or liquid seed media. Slants are incubated at 25°C. in the light for 7 days.

B. Tank Fermentation

In order to provide a larger volume of inoculum, 10 ml of incubated first-stage vegetative culture is used to inoculate 400 ml of a second-stage vegetative growth medium having the same composition as that of the vegetative medium. The second-stage medium is incubated in a two-liter wide-mouth Erlenmeyer flask at 25°C. for 24 hours on a shaker rotating through an arc two inches in diameter at 250 rpm.

Incubated second-stage medium (800 ml), prepared as above described, is used to inoculate 100 liters of sterile production medium selected from one of the following:

MEDIUM IV

| Ingredient | Amount |
|---|---|
| $ZnSO_4 \cdot 7H_2O$ | 0.00455 g/L |
| Soluble meat peptone* | 30.5 g/L |
| Soybean meal | 15.5 g/L |
| Tapioca dextrin** | 2.0 g/L |
| Blackstrap molasses | 10.5 g/L |
| Enzymatic hydrolysate of casein*** | 8.5 g/L |
| $Na_2HPO_4$ | 4.5 g/L |
| $MgSO_4 \cdot 7H_2O$ | 5.5 g/L |
| $FeSO_4 \cdot 7H_2O$ | 0.1 g/L |
| Cottonseed oil | 40.0 ml |
| (Antifoam)**** | 1.0 ml |
| Tap water | 1000.0 ml |

(initial pH 6.8-7.0)

*O.M. Peptone, Amber Laboratories, Juneau, Wisc.

**Stadex 11, A.E. Staley Co., Decatur, Ill.

***N-Z-Amine A, Humko Sheffield Chemical, Lyndhurst, N.J.

****P2000, Dow Corning, Midland, Michigan

MEDIUM V

| Ingredient | Amount |
|---|---|
| Glucose | 2.5% |
| Starch | 1.0% |
| Soluble meat peptone* | 1.0% |
| Blackstrap molasses | 1.0% |
| $CaCO_3$ | 0.2% |
| $MgSO_4 \cdot 7H_2O$ | 0.05% |
| Enzymatic hydrolysate of casein** | 0.4% |
| (Antifoam)*** | 0.02% |
| Tap water | q.s. to volume |

*O.M. Peptone

**N-Z-Amine A

***Antifoam "A", Dow Corning

The inoculated production medium is allowed to ferment in a 165-liter fermentation tank at a temperature of 25°C. for about 7 days. The fermentation medium is aerated with sterile air, maintaining the dissolved oxygen level above approximately 50 percent of air saturation.

C. Third-Stage Vegetative Medium

Whenever the fermentation is carried out in tanks larger than those used for 100-liter fermentation, it is recommended that a third-stage vegetative culture be used to seed the larger tank. A preferred third-stage vegetative medium has the following composition:

| Ingredient | Amount |
|---|---|
| Sucrose | 25 g |
| Blackstrap molasses | 25 g |
| Corn-steep liquor | 6 g |
| Enzymatic hydrolysate of casein* | 10 g |
| Malt extract | 10 g |
| $K_2HPO_4$ | 2 g |
| Tap water | 1000 ml |

(initial pH 6.1)

*N-Z-Case

Preparation 3

Separation of the A-42355 Antibiotic Complex

Whole fermentation broth (4127 liters), obtained by the method described in Example 22 using production medium V, is stirred thoroughly with methanol (4280 liters) for one hour and then is filtered, using a filter aid (Hyflo Super-cel, a diatomaceous earth, Johns-Manville Products Corp.). The pH of the filtrate is adjusted to pH 4.0 by the addition of 5 N HCl. The acidified filtrate is extracted twice with equal volumes of chloroform. The chloroform extracts are combined and concentrated under vacuum to a volume of about 20 liters. This concentrate is added to about 200 liters of diethyl ether to precipitate the A-42355 complex. The precipitate is separated by filtration to give 2775 g of the A-42355 complex as a gray-white powder.

## Isolation and Identification of A-30912 Factors

### Preparation 4

Isolation of A-30912 Factor A

The co-pending application of Karl H. Michel entitled RECOVERY PROCESS FOR A-30912 ANTIBIOTICS, Docket X-5477, filed simultaneously herewith this even date, describes the reversed-phase high performance, low pressure liquid chromatography (HPLPLC) using silica gel/$C_{18}$ adsorbent as a preferred method for the final purification of A-30912 factor A.

A-42355 antibiotic complex (1 g), prepared as described in Preparations 2 and 3, is dissolved in 7 ml of methanol:water:acetonitrile (7:2:1). This solution is filtered and introduced onto a 3.7-cm I.D. x 35-cm glass column [Michel-Miller High Performance Low Pressure (HPLPLC) Chromatography Column, Ace Glass Incorporated, Vineland, NJ 08360] packed with LP-1/$C_{18}$ silica gel reversed-phase resin (10-20 microns), prepared as described in Preparation 10, through a loop with the aid of a valve system. The column is packed in methanol:water:acetonitrile (7:2:1) by the slurry-packing procedure described in Preparation 11. An F.M.I. pump with valveless piston design (maximum flow 19.5 ml/minute) is used to move the solvent through the column at a flow rate of 9 ml/minute at ca. 100 psi, collecting fractions every minute. Elution of the antibiotic is monitored at 280 nm by using a UV monitor (ISCO Model UA-5, Instrument Specialist Co., 4700 Superior Ave., Lincoln, Nebraska 68504) with an optical unit (ISCO Type 6).

Preparation 5

Isolation of A-30912 Factor B

A-42355 complex is separated as described in Preparation 3 except that the concentrated chloroform extracts (285 L) are chromatographed over a silica-gel column (150 L of Grace silica-gel, grade 62) at a flow rate of 2 L/min. The column is washed with chloroform (200 L), eluted with acetonitrile (500 L), and then continuously eluted with acetonitrile:water (98:2) at a flow rate of 1 L/min. Fractions having a volume of approximately 200 L are collected and analyzed individually for biological activity. The bioassay is performed by a paper-disc assay on agar plates seeded with Candida albicans. Fractions 77 through 103 (1365 L) are combined and concentrated under vacuum. The concentrated solution (4.5 L) contains a precipitate which is removed by filtration to give 119 g of factor B-enriched A-42355 complex. The filtrate is concentrated to dryness; the residue obtained is redissolved in an appropriate volume of methanol. The methanol solution is added to diethyl ether (10 volumes) to precipitate the factor-B-containing antibiotic complex. This precipitate is also separated by filtration and dried to give an additional 24 g of factor-B-enriched A-42355 complex as a gray powder.

Factor-B-enriched A-42355 complex thus obtained (1.0 g) is dissolved in 8 ml of methanol: water:acetonitrile (7:2:1). This solution is filtered and introduced onto a silica-gel column (3.7-cm I.D. x 33-cm Michel-Miller Column) through a loop with the aid

of a valve system. The column is packed with LP-1/$C_{18}$ silica-gel reversed-phase resin (10-20 microns), prepared as described in Preparation 10, in methanol:water: acetonitrile (7:2:1) through a loop with the aid of a valve system. The slurry packing procedure described in Preparation 11 is used. The solvent is moved through the column at a flow rate of 10 ml/min at *ca*. 100 psi, using an F.M.I. pump with valveless piston design. One fraction is collected every minute. Elution of the antibiotic is monitored using a UV monitor at 280 nm as in Preparation 15. Fractions 102-110 are combined and concentrated under vacuum to give an oil. The oil is dissolved in a small volume of *tert*-butanol and lyophilized to give 22 mg of A-30912 factor B.

## Preparation 6

### Isolation of A-30912 Factor D

Concentrated chloroform extracts from two fermentation runs (3800 L and 4007 L) obtained by the method described in Preparation 3 are combined and chromatographed on a silica-gel column (Grace, grade 62). The column is washed with chloroform and then is eluted with acetonitrile and acetonitrile:water (98:2). Fractions having a volume of approximately 200 L are collected and analyzed for biological activity by paper-disc assay on agar seeded with *Candida albicans*. Fractions having activity (850 L) are combined and concentrated under vacuum. The concentrated solution (0.7 L) is added to diethyl ether (10 volumes) to precipitate the factor D-enriched A-42355 complex.

This precipitate is removed by filtration and dried to give 32 g, of factor D-enriched A-42355 complex as a gray powder.

Factor D-enriched A-42355 complex thus obtained (1.0 g,) is dissolved in 5 ml. of methanol: water:acetonitrile (7:2:1). This solution is filtered and introduced onto a silica-gel column (3.7-cm I.D. x 30-cm Michel-Miller Column) through a loop with the aid of a valve system. The column is packed with LP-1/$C_{18}$ silica-gel reversed-phase resin (10-20 microns), prepared as described in Preparation 10. Packing is accomplished in methanol:water:acetonitrile (7:2:1) by the slurry-packing procedure described in Preparation 11. The solvent is moved through the column at a flow rate of 8 ml/min at ca. 45 psi using an F.M.I. pump with valveless piston design. One fraction is collected every 2 minutes. Elution of the antibiotic is monitored at 280 nm by using a UV monitor (ISCO Model UA-5) with an optical unit (ISCO Type 6). Fractions 96-108 are combined and concentrated under vacuum to give an oil. This oil is dissolved in a small volume of tert-butanol and lyophilized to give 89 mg, of A-30912 factor D.

Preparation 7

Isolation of A-30912 Factor H

A-42355 antibiotic complex (5.0 g), prepared as described in Preparations 2 and 3, is dissolved in 35 ml of methanol:water:acetonitrile (7:2:1); the resulting solution is filtered and introduced onto a

3.7-cm I.D. x 42-cm glass column (Michel-Miller Column) through a loop with the aid of a valve system. The column is packed with LP-1/$C_{18}$ silica gel reversed phase resin (10-20 microns) in methanol:water:acetonitrile (7:2:1) (Preparation 10) as described in Preparation 11. The solvent is moved through the column at a flow rate of 13 ml/min at ca. 120 psi, using an F.M.I. pump with valveless piston design and collecting one fraction every two minutes. Elution of the antibiotic is monitored by UV at 280 nm as described in Preparation 19, Sect. C. Fractions 112-132 are combined with fractions 106-117 from a second similar purification. The combined fractions are concentrated under vacuum to an oil. The oil is dissolved in a small volume of tert-butanol and lyophilized to give 173 mg of crude A-30912 factor H.

The crude A-30912 factor H (150 mg) is dissolved in 8 ml of methanol:water:acetonitrile (7:2:1); the resulting solution is filtered and introduced onto a 2.0-cm I.D. x 32-cm glass column, as described above. The solvent is moved through the column at a flow rate of 8-ml/min at ca. 80 psi collecting one fraction every three minutes. Elution of the antibiotic is monitored at 280 nm. Fractions 17 and 18 are combined and concentrated under vacuum to give an oil. The oil is dissolved in a small volume of tert-butanol and lyophilized to give 29 mg of A-30912 factor H.

Identification of A-30912 Factors

The individual A-30912 factors can be identified by the use of thin-layer chromatography (TLC). The $R_f$ values of A-30912 factors A-G, using silica gel (Merck, Darmstadt) TLC, a benzene:methanol (7:3) solvent system, and *Candida albicans* bioautography are given in Table VII.

Table VII

| A-30912 Factor | $R_f$ Value |
|---|---|
| A | 0.35 |
| B | 0.45 |
| C | 0.54 |
| D | 0.59 |
| E | 0.27 |
| F | 0.18 |
| G | 0.13 |

The approximate $R_f$ values of A-30912 factors A, B, C, D, and H in different solvent systems, using silica gel TLC (Merck-Darmstadt silica gel #60 plates, 20 x 20 cm) and *Candida albicans* bioautography, are given in Table VIII.

TABLE VIII

| A-30912 Factor | $R_f$ Values - Solvent Systems | | | |
|---|---|---|---|---|
| | a | b | c | d |
| Factor A | 0.28 | 0.14 | 0.28 | 0.43 |
| Factor B | 0.39 | 0.21 | 0.42 | 0.47 |
| Factor C | 0.46 | 0.31 | 0.51 | 0.58 |
| Factor D | 0.50 | 0.38 | 0.57 | 0.61 |
| Factor H | 0.42 | 0.27 | 0.36 | 0.53 |

Solvent Systems

a: ethyl acetate:methanol (3:2)

b: ethyl acetate:methanol (7:3)

c: acetonitrile:water (95:5)

d: ethyl acetate:ethanol:acetic acid (40:60:0.25)

A-30912 factors A, B, D and H can also be indentified by analytical HPLPLC using the following conditions:

| | |
|---|---|
| Column: | glass, 0.8 x 15.0 cm |
| Packing: | Nucleosil® 10-$C_{18}$ (Machery-Nagel and Company); packed using slurry-packing procedure of Example 8 |
| Solvent: | methanol:water:acetonitrile (7:2:1) |
| Sample Volume: | 8 mcl |
| Sample Size: | 8 mcg |
| Column Temperature: | ambient |
| Flow Rate: | 1.8 ml/min |
| Pressure: | *ca.* 200 psi |
| Detector: | UV at 222 nm (ISCO Model 1800 Variable Wavelength UV-Visible Absorbance Monitor) |
| Pump: | LDC Duplex Minipump |
| Injection: | loop injection |

The approximate retention times for A-30912 factors A, B, D, and H under these conditions are summarized in Table IX.

Table IX

| A-30912 Factor | Retention Time (seconds) |
|---|---|
| A | 792 |
| B | 870 |
| H | 990 |
| D | 1,140 |

Preparation 8

Preparation of Antibiotic S31794/F-1

Antibiotic S31794/F-1 is produced by submerged culture of Acrophialophora limonispora NRRL 8095 with stirring, shaking, and/or aeration at pH 3-8, preferably pH 5-7, and at 15-30°C., preferably at 18-27°C., for from 48 to 360 hours, preferably from 120 to 288 hours.

Antibiotic S31794/F-1 is isolated by treating the culture broth (90 L) with ethyl acetate:isopropanol (4:1, 90 L) and homogenizing for 30 minutes at room temperature. The organic phase is separated and evaporated under vacuum at about 40°C. The residue thus obtained is chromatographed on a 10-fold amount of silica gel, using $CHCl_3$:$CH_3OH$ (95:5 to 60:40). Fractions which have antifungal activity are combined and chromatographed on a 100-fold amount of "Sephadex LH-20" with methanol. Fractions from the Sephadex column which have antifungal activity are combined and rechromatographed on a 100-fold amount of silica gel (0.05-0.2 mm) with a $CHCl_3$:$CH_3OH$:$H_2O$ (71:25:4) solvent system. The fractions eluted which have antifungal

activity are combined and evaporated under vacuum to give crude antibiotic S31794/F-1. This product is dissolved in small amounts of methanol and precipitated with diethyl ether to give S31794/F-1 as a white amorphous powder, mp 178-180°C. (dec.) after drying in high vacuum at 25-30°C. Crystallization from a 10-fold amount of ethyl acetate:methanol:water (80:12:8) gives crystalline S31794/F-1, mp 181-183°C. (dec) after drying in high vacuum at 20°C.

## Preparation 9

### Isolation of Antibiotic S31794/F-1

Crude antibiotic S31794/F-1, obtained as described in Preparation 8 after chromatography over Sephadex, is introduced onto a silica-gel column (Michel-Miller Column) through a loop with the aid of a valve system. The column is packed with LP-1/$C_{18}$ silica-gel reversed-phase resin (10-20 microns), prepared as described in Preparation 10, in chloroform: methanol:water (71:25:4) through a loop with the aid of a valve system. The slurry packing procedure described in-Preparation 11 is used. The solvent is moved through the column using an F.M.I. pump with valveless piston design. Elution of the antibiotic is monitored using a UV monitor at 280 nm as in Preparation 22. Fractions having antifungal activity are combined and concentrated under vacuum to give antibiotic S31794/F-1.

S31794/F-1 has $R_f$ values as follow on silica-gel thin-layer chromatography (Merck, 0.25 mm):

| Solvent System | $R_f$ Value |
|---|---|
| Chloroform:methanol:water (71:25:4) | 0.17 |
| Chloroform:methanol:conc. acetic acid (70:29:1) | 0.19 |
| Chloroform:methanol (2:1) | 0.27 |

S31794/F-1 can also be detected by iodine vapor.

## Preparation 10

## Preparation of Silica Gel/$C_{18}$ Reversed Phase Resin

### Step 1: Hydrolysis

LP-1 silica gel (1000 g from Quantum Corp., now Whatman) is added to a mixture of concentrated sulfuric acid (1650 ml) and concentrated nitric acid (1650 ml) in a 5-L round-bottom flask and shaken for proper suspension. The mixture is heated on a steam bath overnight (16 hours) with a water-jacketed condenser attached to the flask.

The mixture is cooled in an ice bath and carefully filtered using a sintered-glass funnel. The silica gel is washed with deionized water until the pH is neutral. The silica gel is then washed with acetone (4 L) and dried under vacuum at 100°C. for 2 days.

Step 2: First Silylation

The dry silica gel from Step 1 is transferred to a round-bottom flask and suspended in toluene (3.5 L). The flask is heated on a steam bath for 2 hours to azeotrope off some residual water. Octadecyltrichlorosilane (321 ml, Aldrich Chemical Company) is added, and the reaction mixture is refluxed overnight (16 hours) with slow mechanical stirring at about 60°C. Care is taken so that the stirrer does not reach near the bottom of the flask. This is to prevent grinding the silica gel particles.

The mixture is allowed to cool. The silanized silica gel is collected, washed with toluene (3 L) and acetone (3 L), and then air-dried overnight (16-20 hours). The dried silica gel is suspended in 3.5 L of acetonitrile:water (1:1) in a 5-L flask, stirred carefully at room temperature for 2 hours, filtered, washed with acetone (3 L) and air-dried overnight.

Step 3: Second Silylation

The procedure from the first silylation is repeated using 200 ml of octadecyltrichlorosilane. The suspension is refluxed at 60°C. for 2 hours while stirring carefully. The final product is recovered by filtration, washed with toluene (3 L) and methanol (6 L), and then dried under vacuum at 50°C. overnight (16-20 hours).

## Preparation 11

### Slurry Packing Procedure for Michel-Miller Columns

### General Information

This procedure is employed for packing silica gel $C_{18}$ reversed phase resin such as that prepared by the method of Preparation 10.

Generally, a pressure of less than 200 psi and flow rates between 5-40 ml/minute are required for this slurry packing technique; this is dependent on column volume and size. Packing pressure should exceed the pressure used during actual separation by 30-50 psi; this will assure no further compression of the adsorbent during separation runs.

A sudden decrease in pressure may cause cracks or channels to form in the packing material, which would greatly reduce column efficiency. Therefore, it is important to let the pressure drop slowly to zero whenever the pump is turned off.

The approximate volume of columns (Ace Glass Cat. No., unpacked) are No. 5795-04, 12 ml; No. 5795-10, 110 ml; No. 5795-16, 300 ml; No. 5795-24, 635 ml; and No. 5796-34, 34 ml.

The time required to pack a glass column will vary from minutes to several hours depending on column size and the experience of the scientist.

### Steps:

1. Connect glass column to a reservoir column via coupling (volume of reservoir column should be twice that of the column). Place both columns in vertical positions (reservoir column above).

2. Weigh out packing material (ca. 100 g for 200 ml column).

3. Add ca. five volumes of solvent to packing material; use a mixture of 70-80% methanol and 20-30% water.

4. Shake well until all particles are wetted, let stand overnight or longer to assure complete soaking of particles by solvent. Decant supernatant liquid.

5. Slurry the resin with sufficient solvent to fill reservoir column. Pour swiftly into reservoir. The column must be pre-filled with the same solvent and the reservoir column should be partly filled with solvent before slurry is poured. The use of larger slurry volumes may also provide good results; however, this will require (a) larger reservoir or (b) multiple reservoir fillings during the packing procedure.

6. Close reservoir with the Teflon plug beneath the column (see Figure 1 of U.S. Patent 4,131,547, plug No. 3); connect to pump; and immediately start pumping solvent through system at maximum flow rate if Ace Cat. No. 13265-25 Pump or similar solvent-delivery system is used (ca. 20 ml/minute).

7. Continue until column is completely filled with adsorbent. Pressure should not exceed maximum tolerance of column during this operation (ca. 200 psi for large columns and 300 psi for analytical columns). In most cases, pressures less than 200 psi will be sufficient.

8. Should pressure exceed maximum values, reduce flow-rate; pressure will drop.

9. After column has been filled with adsorbent, turn off pump; let pressure drop to zero; disconnect reservoir; replace reservoir with a pre-column; fill pre-column with solvent and small amount of adsorbent; and pump at maximum pressure until column is completely packed. For additional information, see general procedure. Always allow pressure to decrease slowly after turning off pump--this will prevent formation of any cracks or channels in the packing material.

10. Relieve pressure and disconnect pre-column carefully. With small spatula remove a few mm (2-4) of packing from top of column; place 1 or 2 filter(s) in top of column; gently depress to top of packing material, and place Teflon plug on top of column until seal is confirmed. Connect column to pump, put pressure on (usually less than 200 psi) and observe through glass wall on top of column if resin is packing any further. If packing material should continue to settle (this may be the case with larger columns), some dead space or channelling will appear and step 9 should be repeated.

## Preparation 12

### Preparation of A-30912A Nucleus

### A. Deacylation of Antibiotic A-30912 Factor A

A fermentation of A. utahensis is carried out as described in Preparation 1, using slant medium A and production medium I and incubating the production medium for about 42 hours. A-30912 factor A (340 g. of crude substrate which contained about 19.7 g. of A-30912 factor A, dissolved in 1.5 L ethanol) is added to the fermentation medium.

Deacylation of A-30912 factor A is monitored by assay against *Candida albicans*. The fermentation is allowed to continue until deacylation is complete as indicated by disappearance of activity vs. *C. albicans*.

B. Isolation of A-30912A Nucleus

Whole fermentation broth (100 liters), obtained as described in Sect. A and containing nucleus from about 20 g of A-30912 factor A, is filtered. The mycelial cake is discarded. The clear filtrate thus obtained (about 93 liters) is passed through a column containing 4.5 liters of HP-20 resin (DIAION High Porous Polymer, HP-Series, Mitsubishi Chemical Industries Limited, Tokyo, Japan) at a rate of 200 ml/minute. The effluent thus obtained is discarded. The column is then washed with up to eight column volumes of deionized water at pH 6.5-7.5 to remove residual filtered broth. This wash water is discarded. The column is then eluted with a water:methanol (7:3) solution (85 liters) at a rate of 200-300 ml/minute.

Elution is monitored using the following procedure: Two aliquots are taken from each eluted fraction. One of the aliquots is concentrated to a small volume and is treated with an acid chloride such as myristoyl chloride. This product and the other (untreated) aliquot are assayed for activity against *Candida albicans*. If the untreated aliquot does not have activity and the acylated aliquot does have activity, the fraction contains A-30912A nucleus. The eluate containing the A-30912A nucleus is concentrated under vacuum to a small volume and lyophilized to give approximately 97 grams of crude nucleus.

C. Purification of A-30912A Nucleus by Reversed-Phase Liquid Chromatography

Crude A-30912A nucleus (25 grams), obtained as described in Section C, is dissolved in 300 ml of water:acetonitrile:acetic acid:pyridine (96:2:1:1). This solution is chromatographed on a 4-liter stainless-steel column (8 cm x 80 cm) filled with Lichroprep RP-18, particle size 25-40 microns (MC/B Manufacturing Chemists, Inc. E/M, Cincinnati, OH). The column is part of a Chromatospac Prep 100 unit (Jobin Yvon, 16-18 Rue du Canal 91160 Longjumeau, France). The column is operated at a pressure of 90-100 psi, giving a flow rate of about 60 ml/minute, using the same solvent. Separation is monitored at 280 nm using a UV monitor (ISCO Absorption Monitor Model UA-5, Instrumention Specialties Co., 4700 Superior Ave., Lincoln, Nebraska 68504) with an optical unit (ISCO Type 6). Fractions having a volume of about 500 ml are collected each minute.

On the basis of absorption at 280 nm, fractions containing A-30912A nucleus are combined, evaporated under vacuum and lyophilized to give 2.6 grams of nucleus. The amount of solvent required to complete this chromatographic separation process varies from 7-8 liters.

D. Characteristics of A30912A nucleus

(a) Empirical formula: $C_{34}H_{51}N_7O_{15}$.

(b) Molecular weight: 797.83.

(c) White amorphous solid, soluble in water, dimethylformamide, dimethylsulfoxide, and methanol; insoluble in chloroform, toluene, and diethylether.

(d) Infrared absorption spectrum (KBr disc.)

Shows absorption maxima at:

3340 broad (OH, H-bonded); 2970, 2930, and 2890 (CH stretch, aliphatic $CH_3$, $CH_2$, CH groups) 1660 and 1625 (several carbonyls C=O); 1510-1550; 1430-1450 (CH wag); 1310-1340; 1230-1260; 1080; 835, 650 broad, and 550 broad $cm^{-1}$.

(e) Electrometric titration in 66% aqueous dimethylformamide indicates the presence of a titratable group with a $pK_a$ value of about 7.35 (initial pH 7.32).

(f) HPLC retention time (K'):11.52 min. under following conditions.

Column: 4 x 300 mm
Packing: silica gel/$C_{18}$
Solvent: ammonium acetate:acetonitrile: water (1:2:97)
Flow Rate: 3 ml/min
Pressure: 2500 psi
Detector: variable wavelength UV at 230 nm
Sensitivity: 0-0.4 A.U.F.S.

Preparation 13

A-30912A nucleus is prepared and purified by the method of Preparation 12 except that tetrahydro-A-30912A is used as the substrate.

Preparation 14

A-30912A nucleus is prepared and purified by the method of Preparation 12 except that aculeacin A is used as the substrate.

Preparation 15

Preparation of A-30912B Nucleus

A. Deacylation of Antibiotic A-30912 Factor B

A fermentation of A. utahensis is carried out as described in Preparation 1, using production medium I. After the culture is incubated for about 48 hours, A-30912 factor B, dissolved in a small amount of methanol, is added to the fermentation medium.

Deacylation of A-30912 factor B is monitored by paper-disc assay against Candida albicans or Neurospora crassa. The fermentation is allowed to continue until deacylation is complete as indicated by disappearance of activity.

B. Isolation of A-30912B Nucleus

Whole fermentation broth, obtained as described in Sect. A is filtered. The mycelial cake is discarded. The clear filtrate thus obtained is passed through a column containing HP-20 resin (DIAION High Porous Polymer, HP-Series, Mitsubishi Chemical Industries

Limited, Tokyo, Japan). The effluent thus obtained is discarded. The column is then washed with up to eight column volumes of deionized water at pH 6.5-7.5 to remove residual filtered broth. This wash water is discarded. The column is then eluted with a water: methanol (7:3) solution. Elution is monitored using the following procedure: Two aliquots are taken from each eluted fraction. One of the aliquots is concentrated to a small volume and is treated with an acid chloride such as myristoyl chloride. This product and the other (untreated) aliquot are assayed for activity against *Candida albicans*. If the untreated aliquot does not have activity and the acylated aliquot does have activity, the fraction contains A-30912B nucleus. The eluate containing A-30912B nucleus is concentrated under vacuum to a small volume and lyophilized to give crude nucleus.

C. Purification of A-30912B Nucleus by Reversed-Phase Liquid Chromatography

Crude A-30912B nucleus, obtained as described in Section C, is dissolved in water:acetonitrile:acetic acid:pyridine (96:2:1:1). This solution is chromatographed on a column filled with Lichroprep RP-18, particle size 25-40 microns (MC/B Manufacturing Chemists, Inc. E/M, Cincinnati, OH). The column is part of a Chromatospac Prep 100 unit (Jobin Yvon, 16-18 Rue du Canal 91160 Longjumeau, France). The column is operated at a pressure of 90-100 psi, giving a flow rate of about 60 ml/minute, using the same solvent. Separation is monitored at 280 nm using a UV monitor (ISCO Absorption

Monitor Model UA-5, Instrumentation Specialties Co., 4700 Superior Ave., Lincoln, Nebraska 68504) with an optical unit (ISCO Type 6).

On the basis of absorption at 280 nm, fractions containing A-30912B nucleus are combined, evaporated under vacuum and lyophilized to give purified A-30912B nucleus.

Preparation 16

A-30912B nucleus is prepared and purified by the method of Preparation 15 except that tetrahydro-A-30912B is used as the substrate.

Preparation 17

Preparation of A-30912D Nucleus

A. Deacylation of A-30912 Factor D

A fermentation of *A. utahensis* is carried out as described in Preparation 1, using production medium I. After the culture is incubated for about 48 hours, A-30912 factor D, dissolved in a small amount of methanol, is added to the fermentation medium.

Deacylation of A-30912 factor D is monitored by paper-disc assay against *Candida albicans* or *Neurospora crassa*. The fermentation is allowed to continue until deacylation is complete as indicated by disappearance of activity.

B. Isolation of A-30912D Nucleus

Whole fermentation broth, obtained as described in Sect. A is filtered. The mycelial cake is discarded. The clear filtrate thus obtained is passed through a

column containing HP-20 resin (DIAION High Porous Polymer, HP-Series, Mitsubishi Chemical Industries Limited, Tokyo, Japan). The effluent thus obtained is discarded. The column is then washed with up to eight column volumes of deionized water at pH 6.5-7.5 to remove residual filtered broth. This wash water is discarded. The column is then eluted with a water: methanol (7:3) solution. Elution is monitored using the following procedure: Two aliquots are taken from each eluted fraction. One of the aliquots is concentrated to a small volume and is treated with an acid chloride such as myristoyl chloride. This product and the other (untreated) aliquot are assayed for activity against Candida albicans. If the untreated aliquot does not have activity and the acylated aliquot does have activity, the fraction contains A-30912D nucleus. The eluate containing A-30912D nucleus is concentrated under vacuum to a small volume and lyophilized to give crude nucleus.

C. Purification of A-30912D Nucleus by Reversed-Phase Liquid Chromatography

Crude A-30912D nucleus, obtained as described in Section C, is dissolved in water:acetonitrile:acetic acid:pyridine (96:2:1:1). This solution is chromatographed on a column filled with Lichroprep RP-18, particle size 25-40 microns (MC/B Manufacturing Chemists, Inc. E/M, Cincinnati, OH). The column is part of a Chromatospac Prep 100 unit (Jobin Yvon, 16-18 Rue du Canal 91160 Longjumeau, France). The column is operated at a pressure of 90-100 psi, giving a flow rate of

about 60 ml/minute, using the same solvent. Separation is monitored at 280 nm using a UV monitor (ISCO Absorption Monitor Model UA-5, Instrumentation Specialties Co., 4700 Superior Ave., Lincoln, Nebraska 68504) with an optical unit (ISCO Type 6).

On the basis of absorption at 280 nm, fractions containing A-30912D nucleus are combined, evaporated under vacuum and lyophilized to give purified A-30912D nucleus.

Preparation 18

A-30912D nucleus is prepared and purified by the method of Preparation 17 except that tetrahydro-A-30912D is used as the substrate.

Preparation 19

Preparation of A-30912H Nucleus

A. Deacylation of Antibiotic A-30912 Factor H

A fermentation of *A. utahensis* is carried out as described in Preparation 1, using production medium I. After the culture is incubated for about 48 hours, A-30912 factor H, dissolved in a small amount of methanol, is added to the fermentation medium.

Deacylation of A-30912 factor H is monitored by paper-disc assay against *Candida albicans* or *Neurospora crassa*. The fermentation is allowed to continue until deacylation is complete as indicated by disappearance of activity.

B. Isolation of A-30912H Nucleus

Whole fermentation broth, obtained as described in Sect. A, is filtered. The mycelial cake is discarded. The clear filtrate thus obtained is passed through a column containing HP-20 resin (DIAION High Porous Polymer, HP-Series, Mitsubishi Chemical Industries Limited, Tokyo, Japan). The effluent thus obtained is discarded. The column is then washed with up to eight column volumes of deionized water at pH 6.5-7.5 to remove residual filtered broth. This wash water is discarded. The column is then eluted with a water:methanol (7:3) solution. Elution is monitored using the following procedure: Two aliquots are taken from each eluted fraction. One of the aliquots is concentrated to a small volume and is treated with an acid chloride such as myristoyl chloride. This product and the other (untreated) aliquot are assayed for activity against *Candida albicans*. If the untreated aliquot does not have activity and the acylated aliquot does have activity, the fraction contains A-30912H nucleus. The eluate containing A-30912H nucleus is concentrated under vacuum to a small volume and lyophilized to give crude nucleus.

C. Purification of A-30912H Nucleus by Reversed-Phase Liquid Chromatography

Crude A-30912H nucleus, obtained as described in Section C, is dissolved in water:acetonitrile:acetic acid:pyridine (96:2:1:1). This solution is chromatographed on a column filled with Lichroprep RP-18, particle size 25-40 microns (MC/B Manufacturing Chemists, Inc. E/M, Cincinnati, OH). The column is part of a Chromatospac Prep 100 unit (Jobin Yvon, 16-18 Rue du Canal 91160 Longjumeau, France). The column is operated at a pressure of 90-100 psi, giving a flow rate of about 60 ml/minute, using the same solvent. Separation is monitored at 280 nm using a UV monitor (ISCO Absorption Monitor Model UA-5, Instrumentation Specialties Co., 4700 Superior Ave., Lincoln, Nebraska 68504) with an optical unit (ISCO Type 6).

Preparation 20

A-30912H nucleus is prepared and purified by the method of Preparation 19 except that tetrahydro-A-30912H is used as the substrate.

Preparation 22

Preparation of S31794/F-1 Nucleus

A. Deacylation of Antibiotic S31794/F-1

A fermentation of *A. utahensis* is carried out as described in Preparation 1, using production medium I. After the culture is incubated for about 48 hours, antibiotic S31794/F-1, dissolved in a small amount of methanol, is added to the fermentation medium.

Deacylation of S31794/F-1 is monitored by paper-disc assay against *Candida albicans*. The fermentation is allowed to continue until deacylation is complete as indicated by disappearance of activity.

B. Isolation of S31794/F-1 Nucleus

Whole fermentation broth, obtained as described in Sect. A is filtered. The mycelial cake is discarded. The clear filtrate thus obtained is passed through a column containing HP-20 resin (DIAION High Porous Polymer, HP-Series, Mitsubishi Chemical Industries Limited, Tokyo, Japan). The effluent thus obtained is discarded. The column is then washed with up to eight column volumes of deionized water at pH 6.5-7.5 to remove residual filtered broth. This wash water is discarded. The column is then eluted with a water: methanol (7:3) solution. Elution is monitored using the following procedure: Two aliquots are taken from each eluted fraction. One of the aliquots is concentrated to a small volume and is treated with an acid chloride such as myristoyl chloride. This product and the other (untreated) aliquot are assayed for activity against Candida albicans. If the untreated aliquot does not have activity and the acylated aliquot does have activity, the fraction contains S31794/F-1 nucleus. The eluate containing S31794/F-1 nucleus is concentrated under vacuum to a small volume and lyophilized to give crude nucleus.

C. Purification of S31794/F-1 Nucleus by Reversed-Phase Liquid Chromatography

Crude S31794/F-1 nucleus, obtained as described in Section B, is dissolved in water:acetonitrile:acetic acid:pyridine (96:2:1:1). This solution is chromatographed on a column filled with

Lichroprep RP-18, particle size 25-40 microns (MC/B Manufacturing Chemists, Inc. E/M, Cincinnati, OH). The column is part of a Chromatospac Prep 100 unit (Jobin Yvon, 16-18 Rue du Canal 91160 Longjumeau, France). The column is operated at a pressure of 90-100 psi, giving a flow rate of about 60 ml/minute, using the same solvent. Separation is monitored at 280 nm using a UV monitor (ISCO Absorption Monitor Model UA-5, Instrumentation Specialties Co., 4700 Superior Ave., Lincoln, Nebraska 68504) with an optical unit (ISCO Type 6).

On the basis of absorption at 280 nm, fractions containing S31794/F-1 nucleus are combined, evaporated under vacuum and lyophilized to give purified S31794/F-1 nucleus.

## Preparation 23

## Preparation of Tetrahydro-A-30912A

A-30912 factor A is dissolved in ethanol. $PtO_2$ in absolute ethanol is reduced to form Pt, which in turn is used to reduce the A-30912 factor A catalytically, using hydrogenation under positive pressure until the reaction is complete (about 2-3 hours). The reaction mixture is filtered and concentrated under vacuum. The residue is dissolved in a small amount of *tert*-butanol and lyophilized to give tetrahydro-A-30912A.

Preparation 24

Preparation of Tetrahydro-A-30912B

A-30912 factor B is dissolved in ethanol. $PtO_2$ in absolute ethanol is reduced to form Pt, which in turn is used to reduce the A-30912 factor B catalytically, using hydrogenation under positive pressure until the reaction is complete (about 2-3 hours). The reaction mixture is filtered and concentrated under vacuum. The residue is dissolved in a small amount of tert-butanol and lyophilized to give tetrahydro-A-30912B.

Preparation 25

Preparation of Tetrahydro-A-30912D

A-30912 factor D is dissolved in ethanol. $PtO_2$ in absolute ethanol is reduced to form Pt, which in turn is used to reduce the A-30912 factor D catalytically, using hydrogenation under positive pressure until the reaction is complete (about 2-3 hours). The reaction mixture is filtered and concentrated under vacuum. The residue is dissolved in a small amount of tert-butanol and lyophilized to give tetrahydro-A-30912D.

Preparation 26

Preparation of Tetrahydro-A-30912H

A-30912 factor H is dissolved in ethanol. $PtO_2$ in absolute ethanol is reduced to form Pt, which in turn is used to reduce the A-30912 factor H catalytically, using hydrogenation under positive pressure until the reaction is complete (about 2-3 hours). The

reaction mixture is filtered and concentrated under vacuum. The residue is dissolved in a small amount of tert-butanol and lyophilized to give tetrahydro-A-30912H.

## Preparations 27-56

Table 1, below, gives the preparation of various N-alkanoyl amino acids. The compounds shown in Table 1 are prepared according to the following general procedure:

The appropriate alkanoic acid chloride is added dropwise to the appropriate amino acid (1:1 mole ratio) dissolved in pyridine. The amount of pyridine employed should be such as to make the concentration of reactants between 0.1 to 0.2M. The solution is stirred at room temperature for about 3 to 6 hours, after which it is poured into a large volume of water. The product precipitates from solution and is collected by filtration and crystallized from methanol.

TABLE 1

Preparation of N-alkanoyl Amino Acids

| Example No. | Alkanoic acid chloride Formula | wt. | Amino Acid Formula | wt. | N-Alkanoyl Amino Acid Formula | wt. |
|---|---|---|---|---|---|---|
| 27 | $CH_3(CH_2)_{10}COCl$ | 3.00 g | $NH_2CH(CH_2C_6H_5)CO_2H$ | 2.0 g. | $CH_3(CH_2)_{10}CONHCH(C_5H_5)CO_2H$ | 2.5 g. |
| 28 | $CH_3(CH_2)_{10}COCl$ | 234 mg | $NH_2(CH)_4CO_2H$ | 482 mg. | $CH_3(CH_2)_{10}CONH(CH_2)_4CO_2H$ | 598 mg. |
| 29 | $CH_3(CH_2)_{10}COCl$ | 21.85 g. | $NH_2(CH_2)_{10}CO_2H$ | 20.1 mg. | $CH_3(CH_2)_{10}CONH(CH_2)_{10}CO_2H$ | 19.97 g. |
| 30 | $CH_3(CH_2)_{10}COCl$ | 11.09 g. | 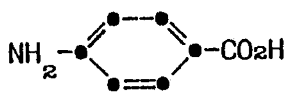 | 6.2 g. | $CH_3(CH_2)_{10}CONH-$ 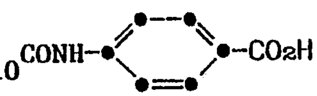 | 6.0 g. |
| 31 | $CH_3(CH_2)_{10}COCl$ | 2.19 g. | 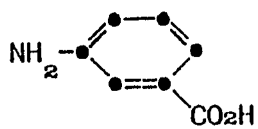 | 1.37 g. | $CH_3(CH_2)_{10}CONH-$ 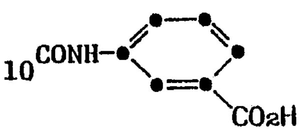 | 3.19 g. |
| 32 | $CH_3(CH_2)_{10}COCl$ | 437 mg. | 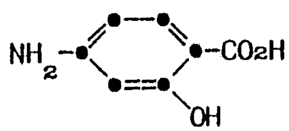 | 306 mg. | $CH_3(CH_2)_{10}CONH-$ 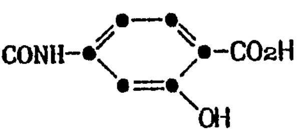 | 670 mg. |
| 33 | $CH_3(CH_2)_{10}COCl$ | 2.17 g. | 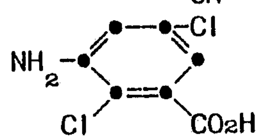 | 2.06 g. | $CH_3(CH_2)_{10}CONH-$ 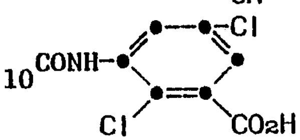 | 3.58 g. |

TABLE 1 (cont.)

Preparation of N-alkanoyl Amino Acids

| Example No. | Alkanoic acid chloride Formula | wt. | Amino Acid Formula | wt. | N-Alkanoyl Amino Acid Formula | wt. |
|---|---|---|---|---|---|---|
| 34 | $CH_3(CH_2)_{10}COCl$ | 2.17 g. | 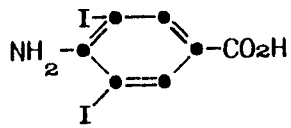 | 3.89 g. | $CH_3(CH_2)_{10}CONH-$ 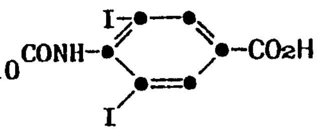 | 5.23 g. |
| 35 | $CH_3(CH_2)_{10}COCl$ | 2.17 g. | 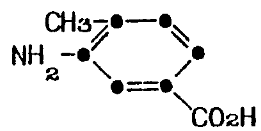 | 1.51 g. | $CH_3(CH_2)_{10}CONH-$ 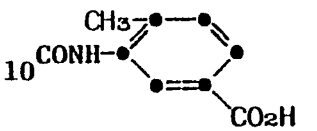 | 3.04 g. |
| 36 | $CH_3(CH_2)_{10}COCl$ | 2.17 g. | 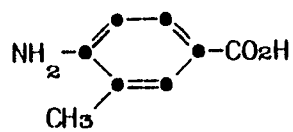 | 1.51 g. | $CH_3(CH_2)_{10}CONH-$ 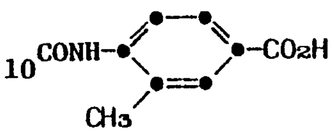 | 2.87 |
| 37 | $CH_3(CH_2)_{10}COCl$ | 2.17 g. | 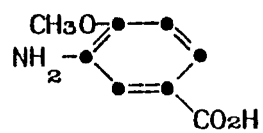 | 1.67 g. | $CH_3(CH_2)_{10}CONH-$ 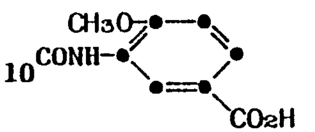 | 3.34 g. |
| 38 | $CH_3(CH_2)_{10}COCl$ | 21.85 g. | 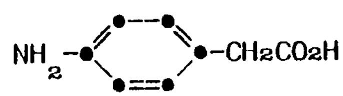 | 15.1 g. | $CH_3(CH_2)_{10}CONH-$ 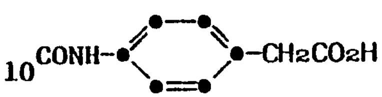 | 33.3 g. |
| 39 | $CH_3(CH_2)_{10}COCl$ | 2.19 g. | 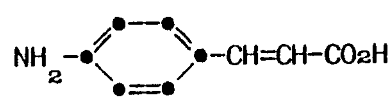 | 1.6 g. | $CH_3(CH_2)_{10}CONH-$ 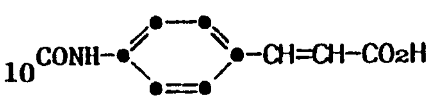 | 2.76 g. |

TABLE 1 (cont.)

Preparation of N-alkanoyl Amino Acids

| Example No. | Alkanoic acid chloride Formula | wt. | Amino Acid Formula | wt. | N-Alkanoyl Amino Acid Formula | wt. |
|---|---|---|---|---|---|---|
| 40 | $CH_3(CH_2)_{10}COCl$ | 21.85 g. | 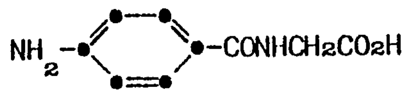 | 19.4 g. | 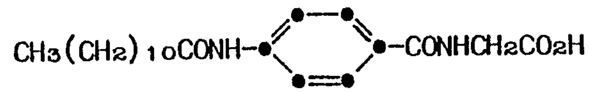 | 37.6 g. |
| 41 | $CH_3(CH_2)_{10}COCl$ | 8.52 g. | 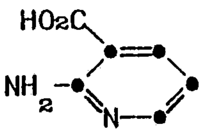 | 5.4 g. | 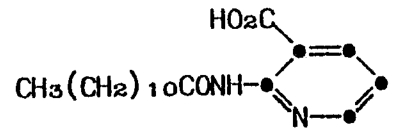 | 7.6 g. |
| 42 | $CH_3(CH_2)_5COCl$ | 14.85 g. | $NH_2(CH_2)_{10}CO_2H$ | 20.1 g. | $CH_3(CH_2)_5CONH(CH_2)_{10}CO_2H$ | 31.3 g. |
| 43 | $CH_3COCl$ | 785 mg. | 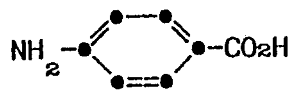 | 1.37 g. | 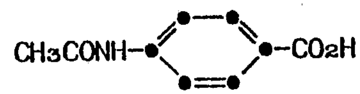 | 1.56 g. |
| 44 | $CH_3(CH_2)_5COCl$ | 1.49 g. | 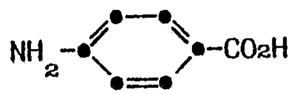 | 1.37 g. | 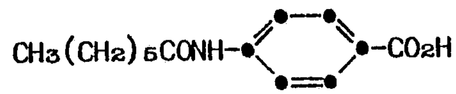 | 2.25 g. |
| 45 | $CH_3(CH_2)_8COCl$ | 1.91 g. | 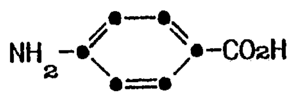 | 1.37 g. | 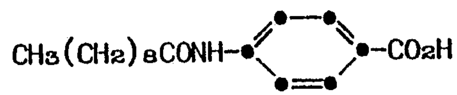 | 2.52 g. |
| 46 | $CH_3(CH_2)_{12}COCl$ | 2.46 g. | 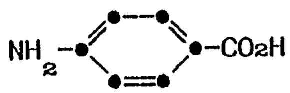 | 1.37 g. | 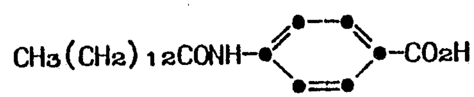 | 2.84 g. |
| 47 | $CH_3(CH_2)_{14}COCl$ | 2.74 g. | 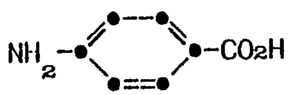 | 1.37 g. | 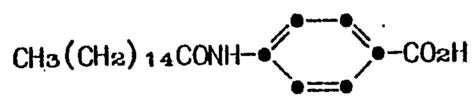 | 3.16 g. |

TABLE 1 (cont.)

Preparation of N-alkanoyl Amino Acids

| Example No. | Alkanoic acid chloride Formula | wt. | Amino Acid Formula | wt. | N-Alkanoyl Amino Acid Formula | wt. |
|---|---|---|---|---|---|---|
| 48 | $CH_3(CH_2)_6COCl$ | 3.42 g. | 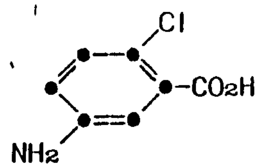 | 3.43 g. | 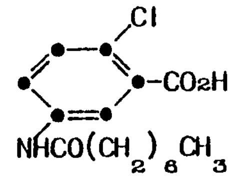 | 3.20 g. |
| 49 | $CH_3(CH_2)_{10}COCl$ | 4.60 g. | 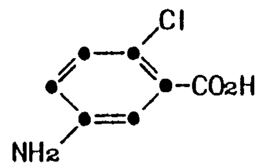 | 3.43 g. | 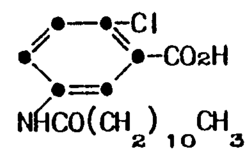 | 2.89 g. |
| 50 | $CH_3(CH_2)_6COCl$ | 3.42 g. | 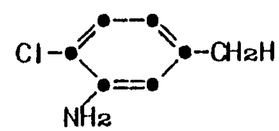 | 3.43 g. | 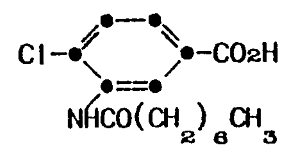 | 3.19 g. |
| 51 | $CH_3(CH_2)_{10}COCl$ | 4.60 g. | 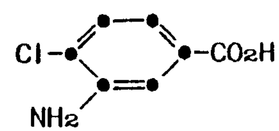 | 3.43 g. | 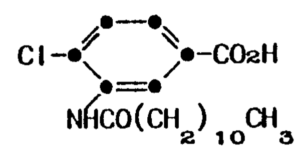 | 4.26 g. |
| 52 | $CH_3(CH_2)_6COCl$ | 3.42 g. | 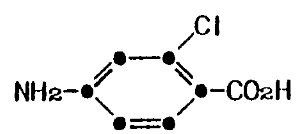 | 3.43 g. | 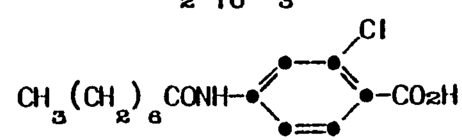 | 4.76 g. |
| 53 | $CH_3(CH_2)_{10}COCl$ | 4.60 g. | 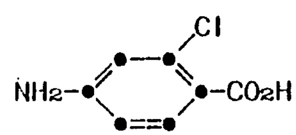 | 3.43 g. | 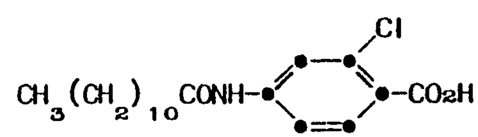 | 6.23 g. |

TABLE 1 (cont.)

Preparation of N-alkanoyl Amino Acids

| Example No. | Alkanoic acid chloride Formula | wt. | Amino Acid Formula | wt. | N-Alkanoyl Amino Acid Formula | wt. |
|---|---|---|---|---|---|---|
| 54 | $CH_3(CH_2)_6COCl$ | 3.42 g. | 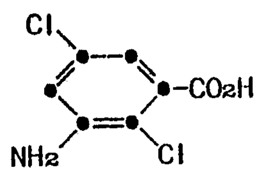 | 6.18 g. | 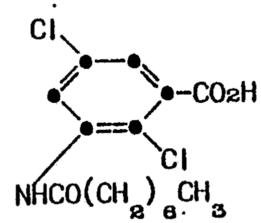 | 4.26 g. |
| 55 | $CH_3(CH_2)_8COCl$ | 4.01 g. | 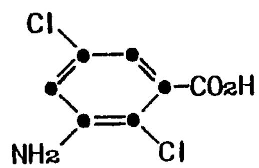 | 4.12 g. | 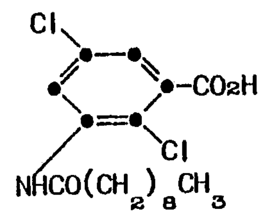 | 3.638 g. |
| 56 | $CH_3(CH_2)_{12}COCl$ | 5.18 g. | 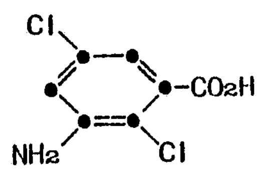 | 4.12 g. | 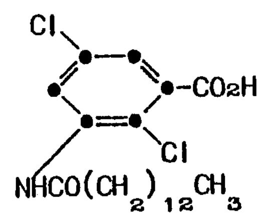 | 4.187 g. |

Preparations 57-86

Table 2, below, gives the preparation of the 2,4,5-trichlorophenyl esters of the N-alkanoyl amino acids shown in Table 1. The compounds set forth in Table 2 are prepared according to the following general procedure:

The N-alkanoylamino acid (1 mole), 2,4,5-trichlorophenol (1.1 mole), and dicyclohexylcarbodiimide (1 mole) are dissolved in methylene chloride, ether or tetrahydrofuran. The solution is stirred at room temperature for about 16 to about 20 hours after which it is filtered. The filtrate is taken to dryness, and the product is crystallized from either acetonitrilewater or diethyl ether-petroleum ether.

TABLE 2

Preparation of 2,4,5-trichlorophenyl esters

| Example No. | N-Alkanoyl Amino Acid Formula | N-Alkanoyl Amino Acid wt | Wt. of 2,4,5-trichlorophenol ester product |
|---|---|---|---|
| 57 | $CH_3(CH_2)_{10}CONHCH(CH_2C_6H_5)CO_2H$ | 333 mg. | 500 mg. |
| 58 | $CH_3(CH_2)_{10}CONH(CH_2)_4CO_2H$ | 598 mg. | 955 mg |
| 59 | $CH_3(CH_2)_{10}CONH(CH_2)_{10}CO_2H$ | 3.83 g. | 1.02 g. |
| 60 | $CH_3(CH_2)_{10}CONH-$ 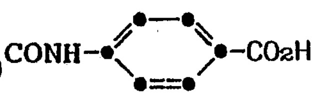 | 638 mg. | 410 mg. |
| 61 | $CH_3(CH_2)_{10}CONH-$ 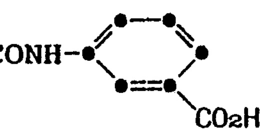 | 3.19 g. | 2.43 g. |
| 62 | $CH_3(CH_2)_{10}CONH-$ 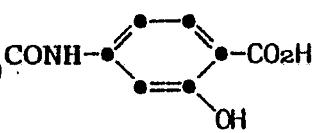 | 670 mg. | 1.03 g. |
| 63 | $CH_3(CH_2)_{10}CONH-$ 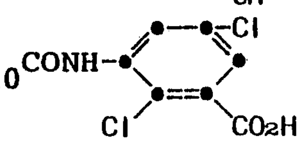 | 3.58 g. | 2.20 g. |
| 64 | $CH_3(CH_2)_{10}CONH-$ 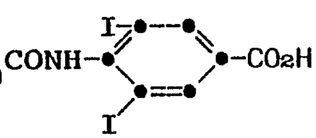 | 5.23 g. | 1.41 g. |

TABLE 2 (cont)

Preparation of 2,4,5-trichlorophenyl esters

| Example No. | N-Alkanoyl Amino Acid Formula | wt | Wt. of 2,4,5-trichlorophenol ester product |
|---|---|---|---|
| 65 | 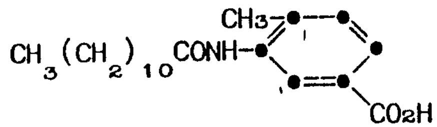 | 3.04 g. | 4.7 g. |
| 66 | 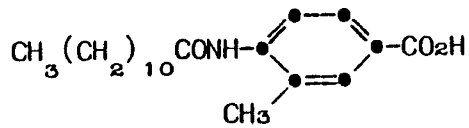 | 2.87 g. | 4.45 g. |
| 67 | 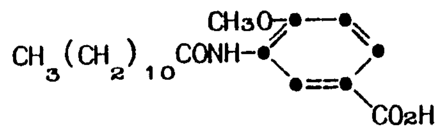 | 3.34 g. | 3.86 g. |
| 68 | 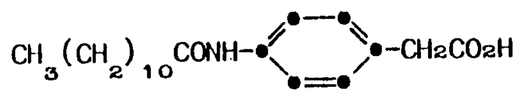 | 3.33 g. | 2.6 g. |
| 69 | 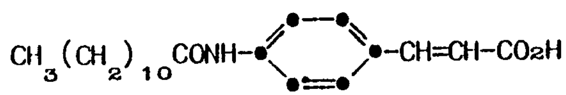 | 2.76 g. | 2.14 g. |
| 70 | 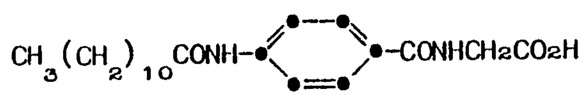 | 3.76 g. | 1.0 g. |
| 71 | 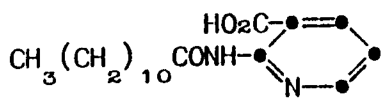 | 3.28 g. | 4.4 g. |

TABLE 2 (cont)

Preparation of 2,4,5-trichlorophenyl esters

| Example No. | N-Alkanoyl Amino Acid Formula | wt | Wt. of 2,4,5-trichlorophenol ester product |
|---|---|---|---|
| 72 | $CH_3(CH_2)_5CONH(CH_2)_{10}CO_2H$ | 4.8 g. | 1.68 g. |
| 73 | 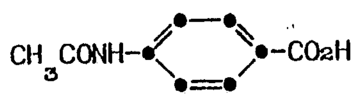 | 895 mg. | 1.48 g. |
| 74 | 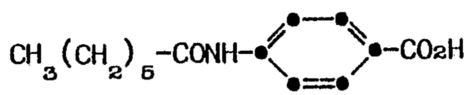 | 1.245 g. | 1.59 g. |
| 75 | 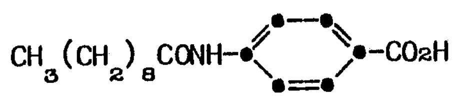 | 2.52 g. | 2.97 g. |
| 76 | 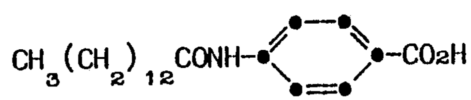 | 2.84 g. | 2.44 g. |
| 77 | 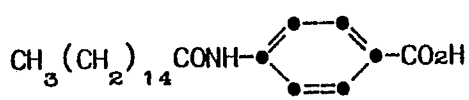 | 3.16 g. | 1.33 g. |
| 78 | 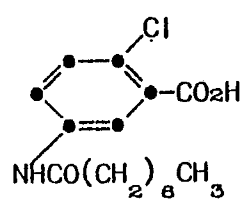 | 2.08 g. | 2.436 g. (700 mg. after recryst.) |

TABLE 2 (cont)

Preparation of 2,4,5-trichlorophenyl esters

| Example No. | N-Alkanoyl Amino Acid Formula | wt | Wt. of 2,4,5-trichlorophenol ester product |
|---|---|---|---|
| 79 | 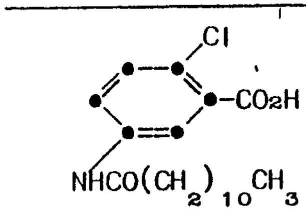 | 2.65 g. | 2.373 g. |
| 80 | 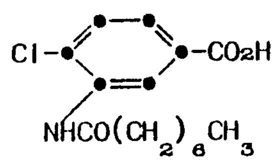 | 2.68 g. | 1.619 g. |
| 81 | 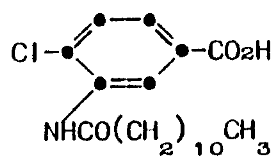 | 3.19 g. | 1.605 g. |
| 82 | 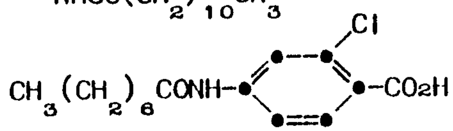 | 2.38 g. | 1.716 g. |
| 83 | 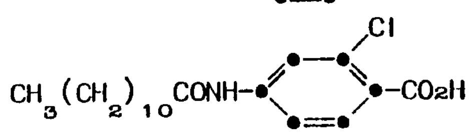 | 2.83 g. | 1.575 g. |

TABLE 2 (cont)

Preparation of 2,4,5-trichlorophenyl esters

| Example No. | N-Alkanoyl Amino Acid Formula | wt | Wt. of 2,4,5-trichlorophenol ester product |
|---|---|---|---|
| 84 | 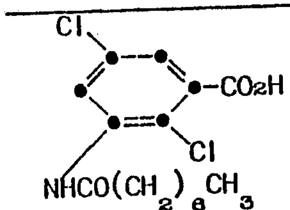 | 4.19 g. | 2.02 g. |
| 85 | 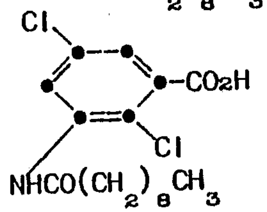 | 2.88 g. | 3.507 g. |
| 86 | 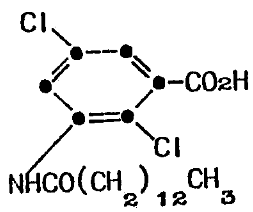 | 3.33 g. | 1.897 g. |

Example 1-29

Table 3, below, gives the preparation of the derivatives of A-30912A nucleus prepared from the N-alkanoyl amino acid 2,4,5-trichlorophenyl esters set forth in Table 2. The compounds set forth in Table 3 are prepared in general according to the following procedure:

To A-30912A nucleus, dissolved in dimethylformamide (DMF) is added the 2,4,5-trichlorophenyl ester of the N-alkanoyl amino acid. The reaction mixture is stirred for 15-18 hours after which it is taken to dryness to give a residue. The residue is washed (two times each) with ethyl ether and by methylene chloride. The washings are discarded. The remaining residue is dissolved in ethyl acetate-methanol (3:2, v/v) and is chromatographed on a silica gel (Woelm 70-150 mesh) column using the aforesaid solvent system as the eluent. The fractions from the chromatograph are monitored by TLC on silica gel (Merck) using ethyl acetate-methanol (3:2, v/v) as the solvent system. Fractions containing the desired product are combined, and solvent is removed to give the product as a residue. The product may be analyzed by reversed phase HPLC as follows: The sample dissolved in $H_2O/CH_3OH/CH_3CN$ (1:2:2 v/v)(1 mg./ml.) is injected into a 1/4 inch by 12 inch stainless steel column packed with $C_{18}$ Micro Bondapak resin (Waters Associates, Inc., Milford, Mass) and the column is eluted with a solvent system comprising $H_2O/CH_3OH/CH_3CN$ (1:2:2 v/v). The elution is

performed at a pressure of 1500 psi with a flow rate of 3 ml./minute using a Waters 600A pump (Waters Associates, Inc.) and chart speed of 0.2 in./minute. Eluent is monitored with a Varian Vari-Chrom UV detector at 230 nm.

The products may also be analyzed by field desorption mass spectrometry (FDMS).

Table 3

N-Alkanoylamino Acid Derivatives of A-30912A Nucleus

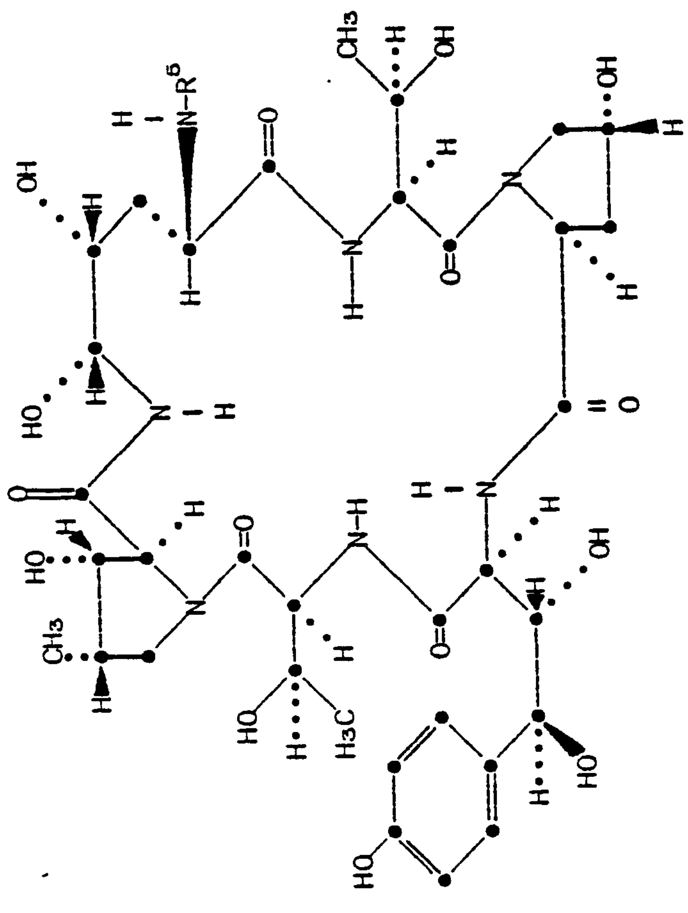

V

TABLE 3

| Example No. | Product: $R^5$ in Formula V | Ester: Example | Ester: Wt. (mg) | A30912A nucleus (mg) | Product (mg) | $M^+$ | HPLC Retention (cm) |
|---|---|---|---|---|---|---|---|
| 1 | $CH_3(CH_2)_{10}CONHCH(CH_2C_6H_5)CO-$ | 31 | 141 | 250 | 158 | 1148 ($M^+$ + 22) | -- |
| 2 | $CH_3(CH_2)_{10}CONH(CH_2)_4-CO-$ | 32 | 795 | 250 | 132 | 1101 ($M^+$ + 22) | -- |
| 3 | $CH_3(CH_2)_{10}CONH(CH_2)_{10}-CO-$ | 33 | 462 | 400 | 327 | 1185 ($M^+$ + 23) | 1.23 |
| 4 | $CH_3(CH_2)_{10}CONH-$ 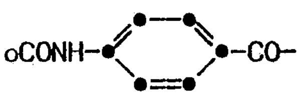 | 34 | 515 | 400 | 247 | 1121 ($M^+$ + 23) | -- |
| 5 | $CH_3(CH_2)_{10}CONH-$ 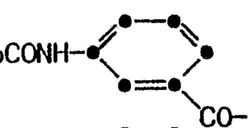 | 35 | 515 | 400 | 302 | 1120 ($M^+$ + 22) | -- |
| 6 | $CH_3(CH_2)_{10}CONH-$ 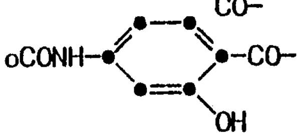 | 36 | 515 | 400 | 354 | 1137 ($M^+$ + 23) | 1.33 |
| 7 | $CH_3(CH_2)_{10}CONH-$ 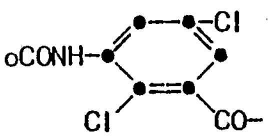 | 37 | 570 | 400 | 196 | 1197 ($M^+$ + 30) | 1.65 |
| 8 | $CH_3(CH_2)_{10}CONH-$ 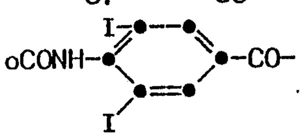 | 38 | 750 | 400 | 291 | -- | 1.20 |

TABLE 3 (cont)

| Example No. | Product: $R^5$ in Formula V | Ester: Example | Ester: Wt. (mg) | A30912A nucleus (mg) | Product (mg) | $M^+$ | HPLC Retention (cm) |
|---|---|---|---|---|---|---|---|
| 9 | 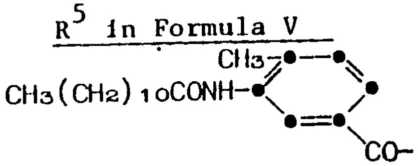 | 39 | 512 | 400 | 182 | 1135($M^+$ + 23) | -- |
| 10 | 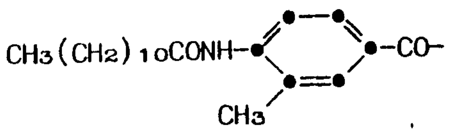 | 40 | 512 | 400 | 166 | 1143($M^+$ + 31) | 1.43 |
| 11 | 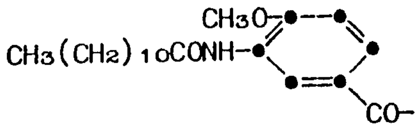 | 41 | 530 | 400 | 120 | 1151($M^+$ + 23) | -- |
| 12 | 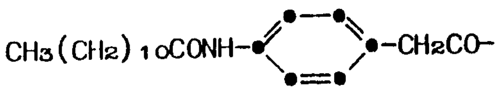 | 42 | 497 | 400 | 452 | 1135($M^+$ + 23) | 1.32 |
| 13 | 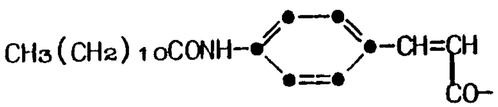 | 43 | 535 | 400 | 286 | 1148($M^+$ + 24) | 1.40 |
| 14 | 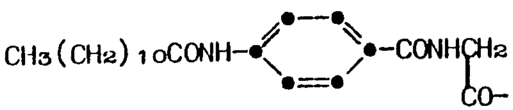 | 44 | 540 | 400 | 453 | 1170($M^+$ + 25) | 1.40 |

TABLE 3 (cont)

| Example No. | Product $R^5$ in Formula V | Ester Example | Ester Wt. (mg) | A30912A nucleus (mg) | Product (mg) | $M^+$ | HPLC Retention (cm) |
|---|---|---|---|---|---|---|---|
| 15 | $CH_3(CH_2)_{10}CONH-$ 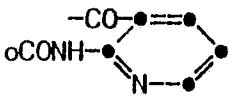 | 45 | 492 | 400 | 277 | -- | -- |
| 16 | $CH_3(CH_2)_6CONH(CH_2)_{10}-CO-$ | 46 | 493 | 400 | 273 | $1115(M^+ + 23)$ | 0.95 |
| 17 | $CH_3CONH-$ 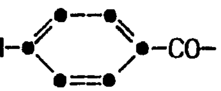 | 47 | 360 | 400 | 213 | $980(M^+ + 22)$ | 1.75 |
| 18 | $CH_3(CH_2)_5-CONH-$ 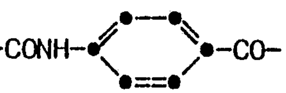 | 48 | 430 | 400 | 218 | -- | -- |
| 19 | $CH_3(CH_2)_8CONH-$ 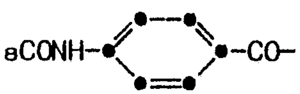 | 49 | 500 | 400 | 162 | $1093(M^+ + 23)$ | 0.90 |
| 20 | $CH_3(CH_2)_{12}CONH-$ 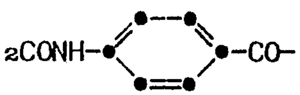 | 50 | 527 | 400 | 234 | $1149(M^+ + 23)$ | 2.35 |
| 21 | $CH_3(CH_2)_{14}CONH-$ 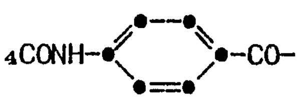 | 51 | 555 | 400 | 350 | $1177(M^+ + 23)$ | 3.23 |
| 22 | 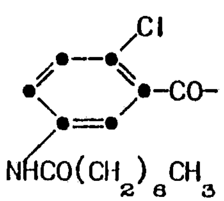 | 52 | 477 | 400 | 176 | $1099(M^+ + 23)$ | 0.7 |

TABLE 3 (cont)

| Example No. | Product $R^5$ in Formula V | Ester Example | Ester Wt.(mg) | A30912A nucleus(mg) | Product(mg) | $M^+$ | HPLC Retention (cm) |
|---|---|---|---|---|---|---|---|
| 23 | 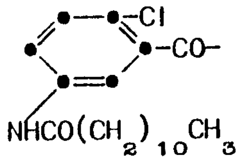 | 53 | 533 | 400 | 127 | 1155($M^+$ + 23) | 2.3 |
| 24 | 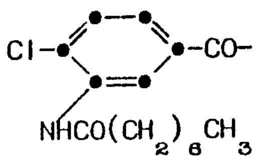 | 54 | 477 | 400 | 319 | 1099($M^+$ + 23) | 1.0 |
| 25 | 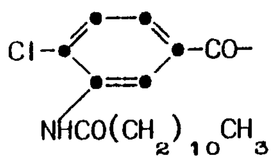 | 55 | 533 | 400 | 214 | 1155($M^+$ + 23) | 3.1 |
| 26 | 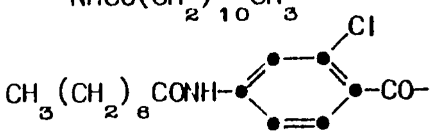 | 56 | 477 | 400 | 290 | | 1.0 |
| 27 | 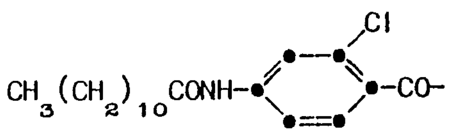 | 57 | 533 | 400 | 325 | | 3.4 |
| 28 | 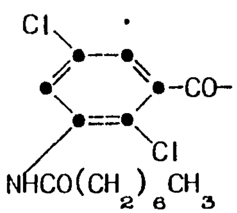 | 58 | 512 | 400 | 324 | | 1.3 |

TABLE 3 (cont)

| Example No. | Product: $R^5$ in Formula V | Ester Example | Ester Wt. (mg) | A30912A nucleus (mg) | Product (mg) | $M^+$ | HPLC Retention (cm) |
|---|---|---|---|---|---|---|---|
| 29 | 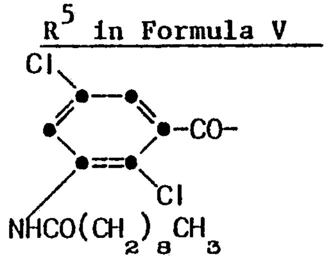 | 59 | 540 | 400 | 281 | 1162($M^+$ + 23) | 1.8 |
| 30 | 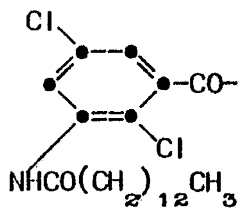 | 60 | 596 | 400 | 269 | 1217($M^+$ + 23) | 7.7 |

## Examples 31-40

Examples 31-40 illustrate the larger-scale preparation of the compounds of Formula III. The specific compounds prepared by the procedures given below are the compounds of Formula III wherein $R^5$ is N-(n-dodecanoyl)-p-aminobenzoyl.

## Example 31

A. Preparation of N-(n-Dodecanoyl)-p-aminobenzoic acid

n-dodecanoyl chloride (8.74 g.; 40 mmoles) is added dropwise to a solution of dissolved p-aminobenzoic acid (5.5 g.; 40 mmoles) dissolved in pyridine (100 ml.). The mixture is stirred for 3 hours and poured into water (3 l.). The precipitate which forms is filtered and dried *in vacuo* to give N-(n-dodecanoyl)-p-aminobenzoic acid (11.01 g.).

B. Preparation of the 2,4,5-trichlorophenyl ester of N-(n-dodecanoyl)-p-aminobenzoic acid

N-(n-Dodecanoyl)-p-aminobenzoic acid (11.01 g.; 34.5 mmole), 2,4,5-trichlorophenol (7.5 g.; 38 mmole), and dicyclohexylcarbodiimide (6.94 g.; 34.5 mmole) are dissolved in methylene chloride (250 ml). The mixture is stirred at room temperature for 3.5 hours and then filtered. The filtrate is evaporated *in vacuo* to give a residue which is crystallized from acetonitrile/water to afford the 2,4,5-trichlorophenyl ester of N-(n-dodecanoyl)-p-aminobenzoic acid (12.84 g.).

C. Acylation of A-30912A nucleus

A-30912A nucleus (8.16 g.; 10.2 mmole) and the 2,4,5-trichlorophenyl ester of N-(n-dodecanoyl)-p-aminobenzoic acid (4.72 g.; 10.2 mmole) are dissolved in dimethylformamide (100 ml.). The solution is stirred at room temperature for 15 hours. Solvent is removed *in vacuo* to give a residue which is washed twice with diethylether. The washes are discarded. The washed residue is dissolved in methanol (50 ml.) and is purified by reversed phase HPLC by means of a "Prep LC/System 500" unit (Waters Associates, Inc., Milford, Mass.) using a Prep Pak-500/$C_{18}$ Column (Waters Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(n-dodecanoyl)-p-aminobenzoyl derivative of A-30912A nucleus (3.5 g).

Example 32

Acylation of A-30912B nucleus

A-30912B nucleus (10.2 mmoles) and the 2,4,5-trichlorophenyl ester of N-(n-dodecanoyl)-p-aminobenzoic acid (prepared as in Example 31 Steps A and B) (10.2 mmoles) are dissolved in dimethylformamide (100 ml.). The solution is stirred at room temperature for 15 hours. Solvent is removed *in vacuo* to give a residue which is washed twice with diethylether. The

washes are discarded. The washed residue is dissolved in methanol (50 ml.) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(n-dodecanoyl)-p-aminobenzoyl derivative of A-30912B nucleus.

## Example 33

The method described in Example 32, with minor changes, can be used to synthesize additional derivatives of the A30912B nucleus. The substitution of the appropriate acyl chloride and amino acid in Step A, the substitution of the appropriate N-alkanoyl amino acid, (plus the use of tetrahydrofuran as the solvent for N-alkanoyl monochloro-substituted aminobenzoic acids), in Step B, and the substitution of the appropriate 2,4,5-trichlorophenyl ester in Example 33 can yield the derivatives of the A30912B nucleus shown below:

N-Alkanoylamino Acid Derivatives of A-30912B Nucleus

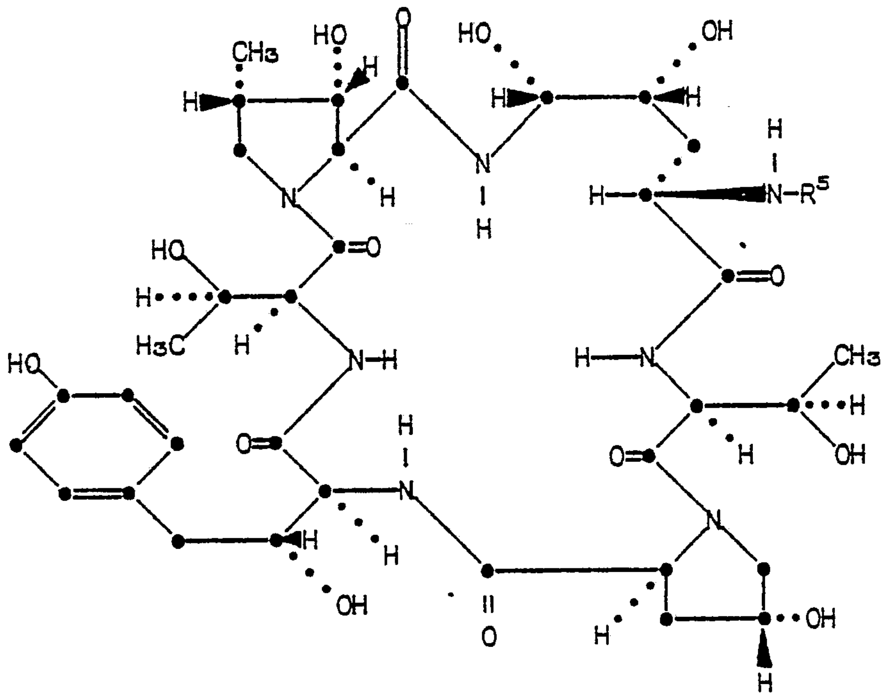

VI

| $R^5$ |
|---|
| $CH_3(CH_2)_{10}CONHCH(CH_2C_6H_5)CO-$ |
| $CH_3(CH_2)_{10}CONH(CH_2)_4-CO-$ |
| $(CH_3(CH_2)_{10}CONH(CH_2)_{10}-CO-$ |
| $CH_3(CH_2)_{10}CONH-$ 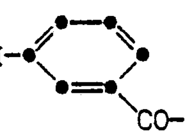 |

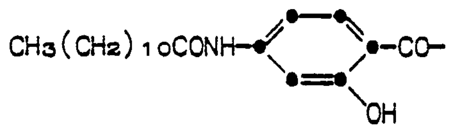

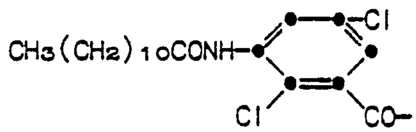

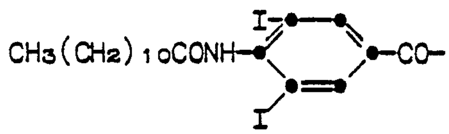

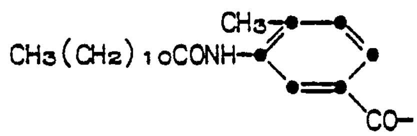

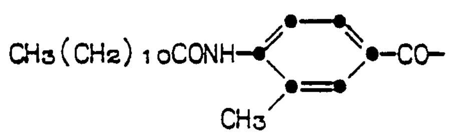

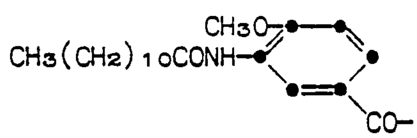

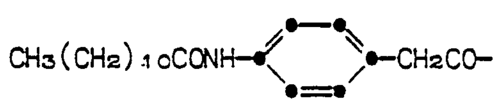

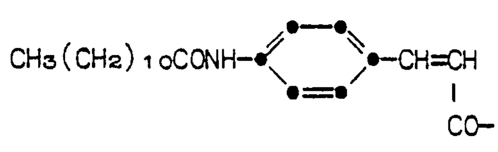

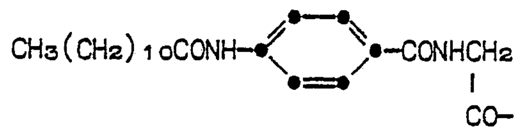

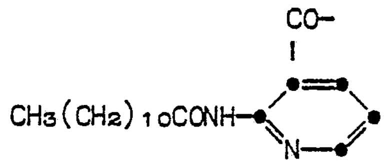

$CH_3(CH_2)_5CONH(CH_2)_{10}-CO-$

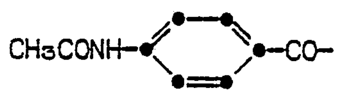

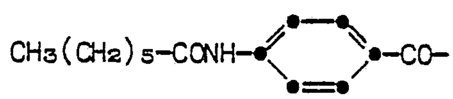

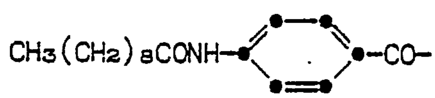

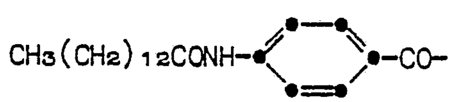

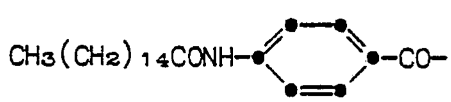

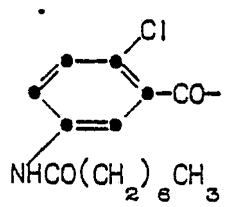

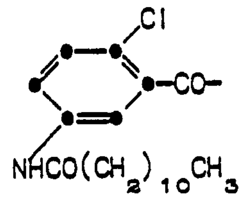

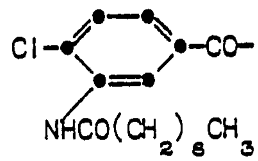

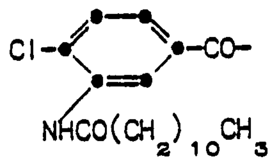

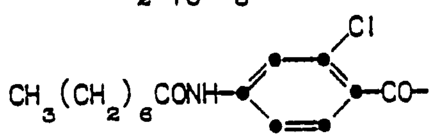

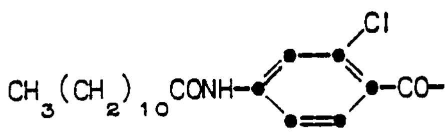

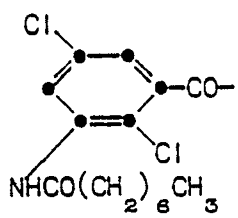

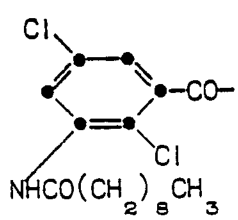

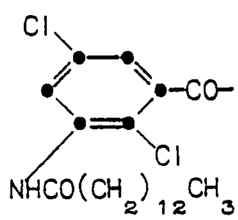

## Example 34

Acylation of A-30912D nucleus

A-30912D nucleus (10.2 mmoles) and the 2,4,5-trichlorophenyl ester of N-(n-dodecanoyl)-p-aminobenzoic acid (prepared as in Example 31 Steps A and B) (10.2 mmoles) are dissolved in dimethylformamide (100 ml.). The solution is stirred at room temperature for 15 hours. Solvent is removed in vacuo to give a residue which is washed twice with diethylether. The washes are discarded. The washed residue is dissolved in methanol (50 ml.) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(n-dodecanoyl)-p-aminobenzoyl derivative of A-30912D nucleus.

## Example 35

The method described in Example 34, with minor changes, can be used to synthesize additional derivatives of the A-30912D nucleus. The substitution of the appropriate acyl chloride and amino acid in Step A, the substitution of the appropriate N-alkanoyl amino acid, (plus the use of tetrahydrofuran as the solvent for N-alkanoyl monochloro-substituted aminobenzoic acids), in Step B, and the substitution of the appropriate 2,4,5-trichlorophenyl ester in Example 34 can yield the derivatives of the A-30912D nucleus shown below wherein $R^5$ is defined the same as in Example 32.

N-Alkanoylamino Acid Derivatives of A-30912D Nucleus

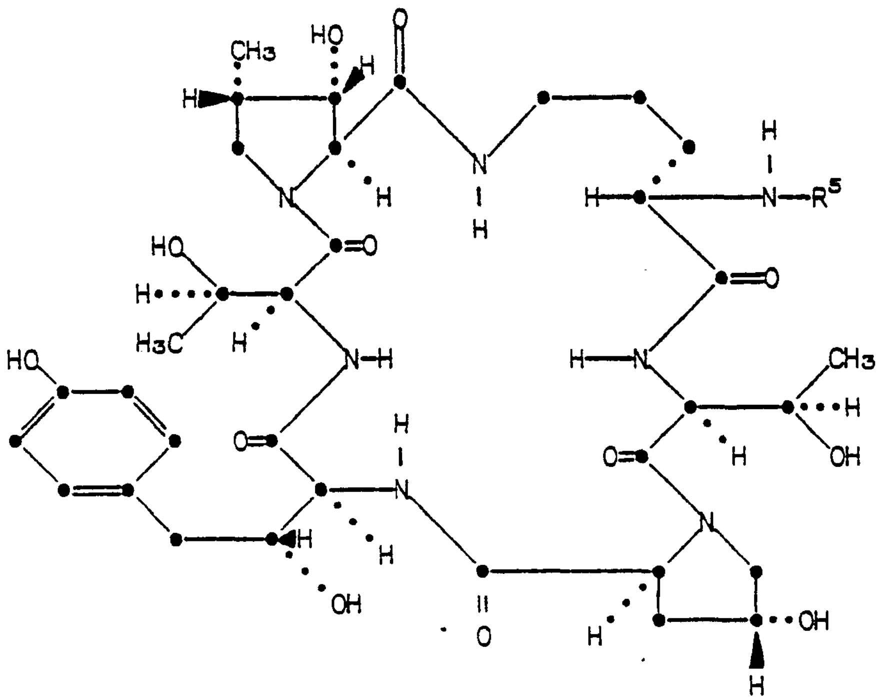

VII

Example 36

Acylation of A-30912H nucleus

A-30912H nucleus (10.2 mmoles) and the 2,4,5-trichlorophenyl ester of N-(n-dodecanoyl)-p-aminobenzoic acid (prepared as in Example 31 Steps A and B) (10.2 mmoles) are dissolved in dimethylformamide (100 ml.).

The solution is stirred at room temperature for 15 hours. Solvent is removed *in vacuo* to give a residue which is washed twice with diethylether. The washes are discarded. The washed residue is dissolved in methanol (50 ml.) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(n-dodecanoyl)-p-aminobenzoyl derivative of A-30912H nucleus.

## Example 37

The method described in Example 36, with minor changes, can be used to synthesize additional derivatives of the A30912H nucleus. The substitution of the appropriate acyl chloride and amino acid in Step A, the substitution of the appropriate N-alkanoyl amino acid, (plus the use of tetrahydrofuran as the solvent for N-alkanoyl monochloro-substituted aminobenzoic acids), in Step B, and the substitution of the appropriate 2,4,5-trichlorophenyl ester in Example 36 can yield the derivatives of the A30912H nucleus shown below wherein $R^5$ is defined the same as in Example 32.

N-Alkanoylamino Acid Derivatives of A-30912H Nucleus

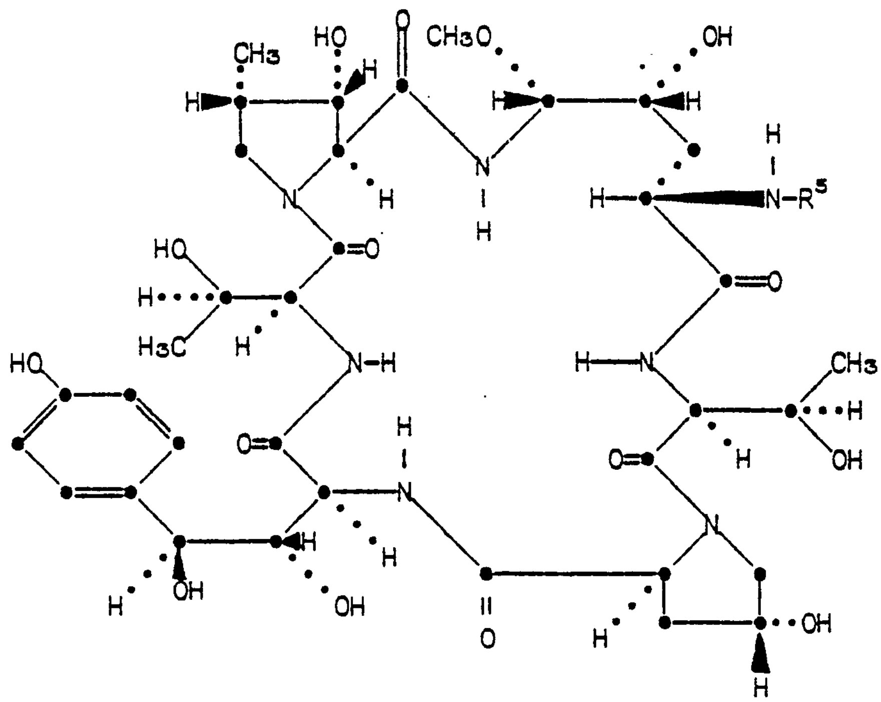

VIII

Example 38

The following procedure illustrates the preparation of the N-(n-dodecanoyl)-p-aminobenzoyl derivative of A-30912H nucleus from A-30912A nucleus.

A-30912A nucleus is treated with 2,4,5-trichlorophenyl N-(n-dodecanoyl)-p-aminobenzoate according to the procedure of Example 36. The derivative thus obtained is methylated by treating a sample (20 mg) with 3% HCl-methanol (0.06 ml) in dimethylformamide. The solution is allowed to stand with stirring for 16 hours afterwhich the solvent is removed under reduced pressure and a residue is obtained. The residue is purified by reversed-phase HPLC using silica gel/$C_{18}$ resin.

Example 39

Acylation of S31794/F-1 nucleus

S31794lF-1 nucleus (10.2 mmoles) and the 2,4,5-trichlorophenyl ester of N-(n-dodecanoyl)-p-aminobenzoic acid (prepared as in Example 31 Steps A and B) (10.2 mmoles) are dissolved in dimethylformamide (100 ml.). The solution is stirred at room temperature for 15 hours. Solvent is removed in vacuo to give a residue which is washed twice with diethylether. The washes are discarded. The washed residue is dissolved in methanol (50 ml.) and is purified by reversed phase HPLC by means of a "Prep LC/System 500" unit (Waters Associates, Inc., Milford, Massachusetts) using a Prep Pak-500/C18 column (Water Associates, Inc.) as the stationary phase. The column is eluted isocratically with $H_2O/CH_3OH/CH_3CN$ (25:65:10 v/v) at 500 psi. The fractions are analyzed by TLC using silica gel plates and $H_2O/CH_3OH/CH_3CN$ (25:65:10 v/v) as the solvent system. Fractions containing the desired product are combined and lyophilized to give the N-(n-dodecanoyl)-p-aminobenzoyl derivative of S31794/F-1 nucleus.

Example 40

The method described in Example 39, with minor changes, can be used to synthesize additional derivatives of the S31794/F-1 nucleus. The substitution of the appropriate acyl chloride and amino acid in Step A, the substitution of the appropriate N-alkanoyl amino acid, (plus the use of tetrahydrofuran as the solvent for N-alkanoyl monochloro-substituted aminobenzoic acids), in Step B, and the substitution of the appropriate 2,4,5-trichlorophenyl ester in Example 39 can yield the derivatives of the S31794/F-1 nucleus shown below where $R^5$ is defined the same as in Example 32.

N-Alkanoylamino Acid Derivatives of S31794/F-1 Nucleus

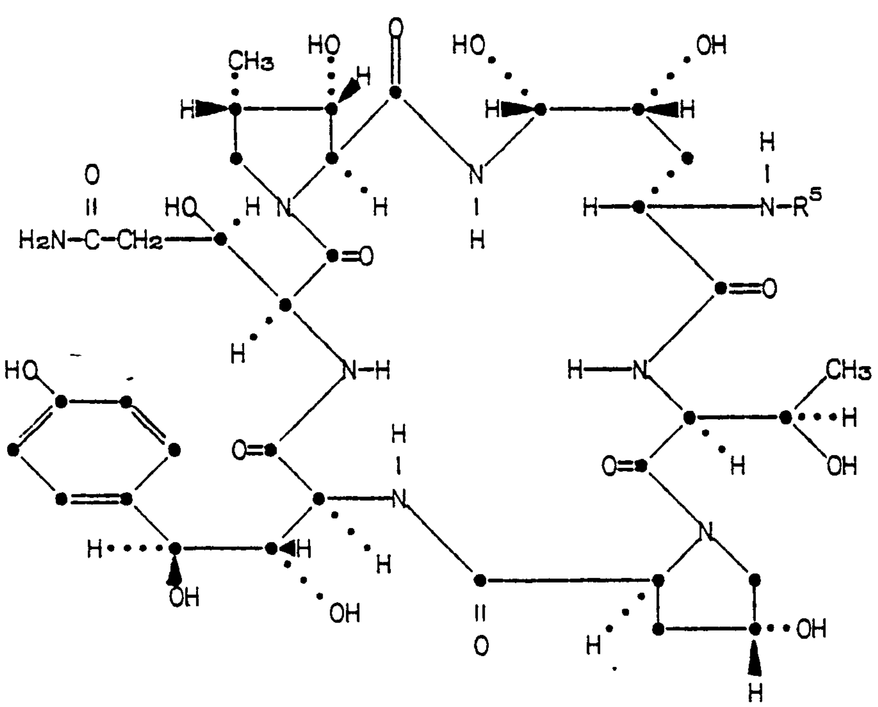

IX

## Example 41

The antifungal activity of the compounds of Formula III can be demonstrated and elicited in vitro in standard disc-diffusion tests and agar-dilution tests, and in vivo in standard tests in mice which assess effectiveness against a systemic fungal infection. The results of the antifungal testing of representative compounds of Formula V (Example 1-30) are set forth in Tables 4, 5, 6 and 7.

Tables 4 and 5 give the results of the testing in vitro of the compounds of Examples 61-81 by agar-plate disc-diffusion methods. In Table 4 activity is measured by the size (diameter in mm.) of the observed zone of inhibition of the microorganism produced by the test compound. In Table 5, activity is measured by the minimal inhibitory concentration (MIC) of the substance (μg/disc) required to inhibit growth of the test organism. Table 6 gives the results of the testing in vitro of the N-(n-dodecanoyl)-p-aminobenzoyl derivative of A30912A nucleus (Formula III, $R^1$ is N-(dodecanoyl)-p-aminobenzoyl) against five strains of Candida albicans by the agar dilution method. In Table 6 activity is measured by the minimal inhibitory concentration (MIC) of the substance (μg/ml) required to inhibit the test organism.

The results of in vivo tests to evaluate the effectiveness of the compound of Examples 61-81, 86 and 88 against an infection caused by Candida albicans A-26 in mice are given in Table 7, where activity is measured by the $ED_{50}$ value (the dose in mg/kg. required

to cure 50% of the test animals). Where an $ED_{50}$ value was not obtained, activity is indicated by the lowest dose at which a significant anti-fungal effect is observed. In this test, groups of male albino mice (specific pathogen free), weighing 18 to 20 grams, are infected intravenously with Candida albicans A-26. The animals are X-irradiated 24 hours prior to infection at about 50 roentgens per minute for 8 minutes (400 total dose) to reduce immune responses to the infecting organism. At 0, 4, and 24 hours post infection each group of mice is given graded doses subcutaneously of the test compound as a suspension in 33% polyethylene glycol-water. The day of death for each animal is recorded. Student's t test statistical comparison of the average day of death is made between each group of infected-treated animals at a particular dosage level and 10 infected-untreated animals to determine if treatment significantly extends survival time.

Table 8 gives the results of the testing of compounds for absorption after oral administration. In this test, mice are gavaged with a dose of 416 mg/kg of the test compound suspended in 33% PEG 400-water. At time intervals, blood samples are taken from the orbital sinus and are assayed for antibiotic activity as follows: A 7 mm. disc containing 20 µl of whole blood is placed on agar seeded with Aspergillus montevidensis A35137. After 40 hours incubation at 30°C. zones of inhibition from the blood samples are compared to a standard obtained from the test compound, and the amount of compound in the blood sample is calculated.

Table 4

Antifungal Activity By the Agar Plate Disc Diffusion Test

| Compound | | Size of Zone of Inhibition (mm)[a] | | | |
|---|---|---|---|---|---|
| Example No. | $R^5$ of Formula V | Saccharomyces pastorianus X-52 | Neurospora Crassa 846 | Trichophyton mentagrophytes A-23 | Candida albicans A-26 |
| 1 | $CH_3(CH_2)_{10}CONHCH(CH_2C_6H_5)-CO-$ | 18 | 42* | 55* | 25 |
| 2 | $CH_3(CH_2)_{10}CONH(CH_2)_4-CO-$ | 15 | 27* | 60* | 25 |
| 3 | $CH_3(CH_2)_{10}CONH(CH_2)_{10}-CO-$ | 15 | 28* | 55* | 24 |
| | | 18 | 35 | 63* | 25. |
| | | 15 | 28 | 56 | 24 |
| 4 | $CH_3(CH_2)_{10}CONH-$ 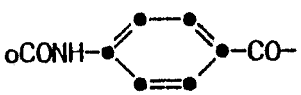 | 21 | 33* | 55* | 23 |
| | | 17 | 35* | 56* | 19 |
| 5 | $CH_3(CH_2)_{10}CONH-$ 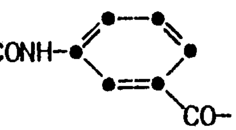 | 17 | -- | -- | 20 |
| 6 | $CH_3(CH_2)_{10}CONH-$ 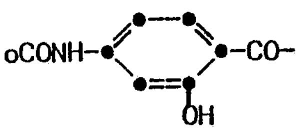 | 18 | -- | -- | 23 |
| 7 | $CH_3(CH_2)_{10}CONH-$ 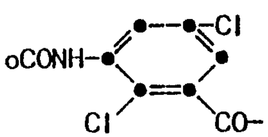 | 19 | 35* | 44* | 27 |

Table 4 (cont)

Antifungal Activity By the Agar Plate Disc Diffusion Test

| Example No. | Compound: $R^5$ of Formula V | Size of Zone of Inhibition (mm)[(a)]: Saccharomyces pastorianus X-52 | Neurospora Crassa 846 | Trichophyton mentagrophytes A-23 | Candida albicans A-26 |
|---|---|---|---|---|---|
| 8 | $CH_3(CH_2)_{10}CONH-$ 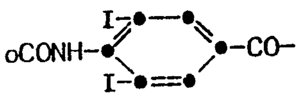 | 17 | 30* | 60 | 25 |
| 9 | $CH_3(CH_2)_{10}CONH-$ 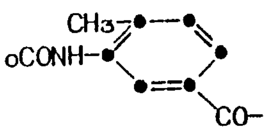 | 17 | 30* | -- | 16 |
| 10 | $CH_2(CH_2)_{10}CONH-$ 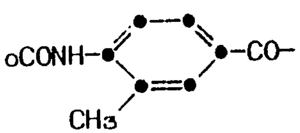 | 19 | 25* | 45* | 26 |
| 11 | $CH_3(CH_2)_{10}CONH-$ 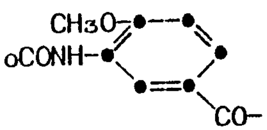 | 10 | 32* | 50* | 20 |
| 12 | $CH_3(CH_2)_{10}CONH-$ 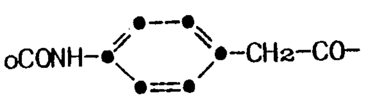 | 20 | 30* | 55* | 19 |
| 13 | $CH_3(CH_2)_{10}CONH-$ 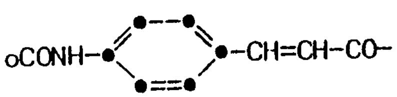 | 17 | 29* | 24* | 60* |

Table 4 (cont)

Antifungal Activity By the Agar Plate Disc Diffusion Test

| Compound | | Size of Zone of Inhibition (mm)[(a)] | | | |
|---|---|---|---|---|---|
| Example No. | $R^5$ of Formula V | Saccharomyces pastorianus X-52 | Neurospora Crassa 846 | Trichophyton mentagrophytes A-23 | Candida albicans A-26 |
| 14 | $CH_3(CH_3)_{10}CONH-$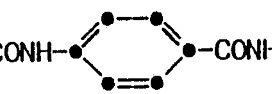$-CONHCH_2-$ | 13 | 28* | -- | 15 |
| 15 | $CH_3(CH_2)_{10}CONH-$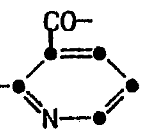 | 14 | 30* | 54* | 24 |
| 16 | $CH_3(CH_2)_5CONH(CH_2)_{10}-CO-$ | -- | 20* | 35* | 10 |
| 17 | $CH_3CONH-$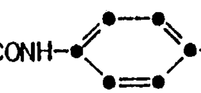$-CO-$ | 17 | 24* | 51* | 24 |
| 18 | $CH_3(CH_2)_5CONH-$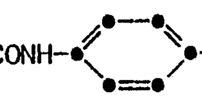$-CO-$ | 20 | 25 | 14 | 45* |
| 19 | $CH_3(CH_2)_8CONH-$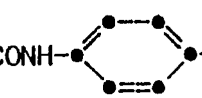$-CO-$ | 17 | 30* | -- | 24 |
| 20 | $CH_3(CH_2)_{12}CONH-$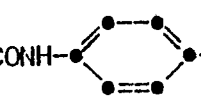$-CO-$ | 17 | -- | -- | 22 |

Table 4 (cont)

Antifungal Activity By the Agar Plate Disc Diffusion Test

| Compound | | Size of Zone of Inhibition (mm)[(a)] | | | |
|---|---|---|---|---|---|
| Example No. | $R^5$ of Formula V | Saccharomyces pastorianus X-52 | Neurospora Crassa 846 | Trichophyton mentagrophytes A-23 | Candida albicans A-26 |
| 21 | $CH_3(CH_2)_{14}CONH$- 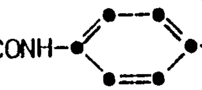 -CO- | 24 | -- | -- | 25 |

(a) Compounds were tested as suspension in methanol. The compounds were tested at a concentration of 1 mg/ml by a dipping 7-mm disc into the suspension and placing it on the agar surface. Incubation: 24-48 hours at 25-37°C.

*Measurable zone of inhibition with regrowth of organism around disc.

Table 5

Antifungal Activity By the Agar Plate Disc Diffusion Test

| Compound | | MIC (μg/disc)* | |
| --- | --- | --- | --- |
| Example No. | $R^5$ or Formula V | Candida albicans A-26 | Trychophyton mentagrophytes #6 |
| 1 | $CH_3(CH_2)_{10}CONHCH(CH_2C_6H_5)-CO-$ | 0.625 | <0.039 |
| 2 | $CH_3(CH_2)_{10}CONH(CH_2)_4-CO-$ | 1.25 | 0.078 |
| 3 | $CH_3(CH_2)_{10}CONH(CH_2)_{10}-CO-$ | 2.5<br>1.25 | 0.156<br>0.156 |
| 4 | $CH_3(CH_2)_{10}CONH-$ 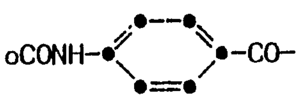 | 0.625<br>1.25 | <0.039<br>0.678 |
| 5 | $CH_3(CH_2)_{10}CONH-$ 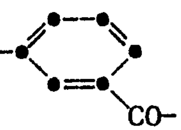 | 5.0 | <0.039 |
| 6 | $CH_3(CH_2)_{10}CONH-$ 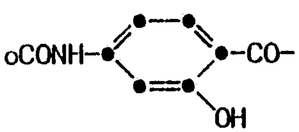 | 1.25 | <0.039 |
| 7 | $CH_3(CH_2)_{10}CONH-$ 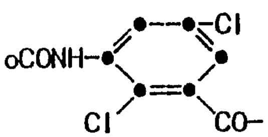 | 0.625 | <0.039 |

Table 5 (cont)

Antifungal Activity By the Agar Plate Disc Diffusion Test

| Compound | | MIC (μg/disc)* | |
|---|---|---|---|
| Example No. | $R^5$ of Formula V | Candida albicans A-26 | Trychophyton mentagrophytes #6 |
| 8 | 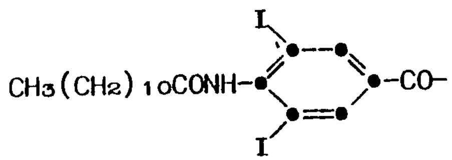 | 1.25 | 0.078 |
| 9 | 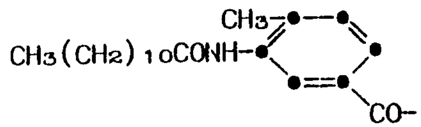 | >20 | <0.039 |
| 10 | 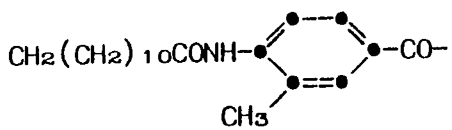 | 20 | <0.039 |
| 11 | 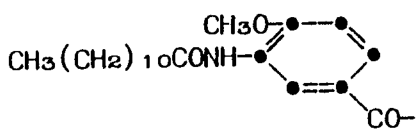 | -- | -- |
| 12 | 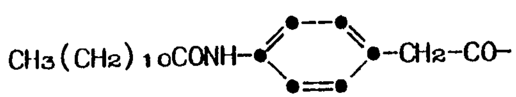 | 2.5 | 0.078 |

Table 5 (cont)

Antifungal Activity By the Agar Plate Disc Diffusion Test

| Compound | | MIC (μg/disc)* | |
|---|---|---|---|
| Example No. | $R^5$ of Formula V | Candida albicans A-26 | Trychophyton mentagrophytes #6 |
| 13 | 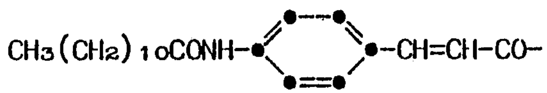 | 1.25 | <0.039 |
| 14 | 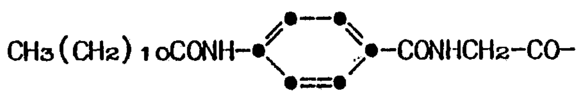 | 10 | 0.078 |
| 15 | 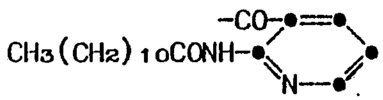 | 2.5 | 0.078 |
| 16 | $CH_3(CH_2)_5CONH(CH_2)_{10}-CO-$ | >20;80 | 0.625 |
| 17 | 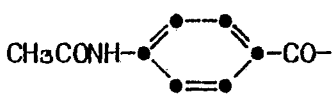 | >20;40 | 0.312 |
| 18 | 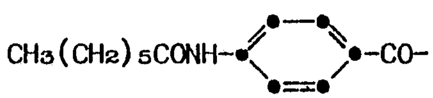 | >20;160 | 1.25 |
| 19 | 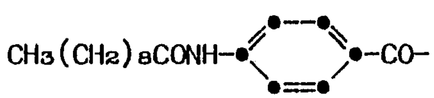 | 0.625 | -- |

Table 5 (cont)

Antifungal Activity By the Agar Plate Disc Diffusion Test

| Compound | | MIC (µg/disc)* | |
|---|---|---|---|
| Example No. | $R^5$ of Formula V | Candida albicans A-26 | Trychophyton mentagrophytes #6 |
| 20 | 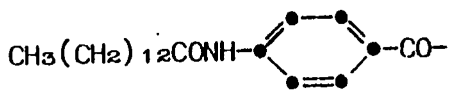 | 1.25 | <0.039 |
| 21 | 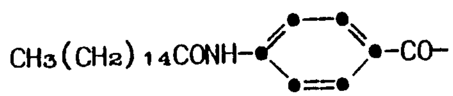 | 0.312 | <0.039 |

*Compounds were suspended in 0.01M sodium borate solution, pH 7.5. The compounds were tested at 20 µg/disc at top level and at two-fold dilutions until end points were reached. Incubation: 24 hours at 30°C.

**Measurable zones of inhibition with regrowth of organism around disc.

Table 6

*In vitro* activity of the N-(n-dodecanoyl)-*p*-aminobenzoyl (Example 4) and the N-(n-dodecanoyl)-5-amino-n-pentanoyl (Example 2) derivatives of A-30912A nucleus against 5 strains of *Candida albicans* by the agar dilution assay.

| | MIC (µg/ml) | | | | |
|---|---|---|---|---|---|
| Compound | A26 | SBH 16 | SBH 31 | SBH 28 | SBH 29 |
| Ex. 4 | 0.312 | 0.625 | 0.625 | 0.625 | 0.625 |
| Ex. 2 | 1.25 | 2.5 | 2.5 | 2.5 | 2.5 |

## Table 7

### Therapeutic Activity Against Candida Albicans A-26 in Mice*

| Example No. | Compound $R^5$ of Formula V | $ED_{50}$ (mg/kg)** | Lowest Active Dose (mg/kg) |
|---|---|---|---|
| 1 | $CH_3(CH_2)_{10}CONHCH(CH_2C_6H_5)-CO-$ | >40 | >40 |
| 2 | $CH_3(CH_2)_{10}CONH(CH_2)_4-CO-$ | 22 | 20 |
| 3 | $CH_3(CH_2)_{10}CONH(CH_2)_{10}-CO-$ | >40 | >40 |
| 4 | $CH_3(CH_2)_{10}CONH-$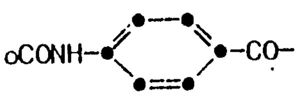 | 15<br>15 | 10<br>≤5 |
| 5 | $CH_3(CH_2)_{10}CONH-$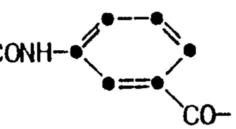 | >40 | >40 |
| 6 | $CH_3(CH_2)_{10}CONH-$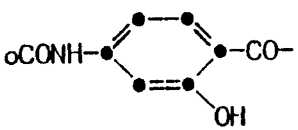 | >40 | 40 |
| 7 | $CH_3(CH_2)_{10}CONH-$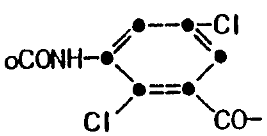 | 14 | 10 |

Table 7 (cont)

Therapeutic Activity Against Candida Albicans A-26 in Mice*

| Example No. | Compound R5 of Formula V | $ED_{50}$ (mg/kg)** | Lowest Active Dose (mg/kg) |
|---|---|---|---|
| 8 | $CH_3(CH_2)_{10}CONH-$ 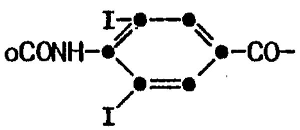 | >40 | 40 |
| 9 | $CH_3(CH_2)_{10}CONH-$ 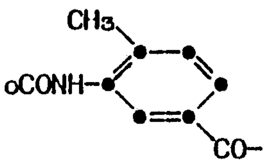 | >40 | >40 |
| 10 | $CH_2(CH_2)_{10}CONH-$ 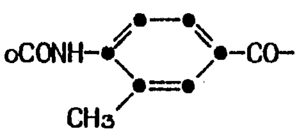 | >40 | >40 |
| 11 | $CH_3(CH_2)_{10}CONH-$ 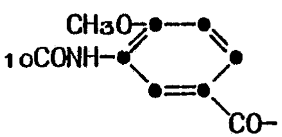 | >40 | >40 |
| 12 | $CH_3(CH_2)_{10}CONH-$ 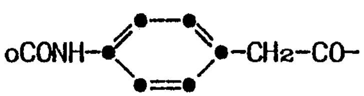 | >40 | 40 |
| 13 | $CH_3(CH_2)_{10}CONH-$ 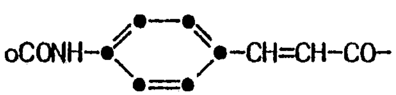 | 24 | 20 |

Table 7 (cont)

Therapeutic Activity Against Candida Albicans A-26 in Mice*

| Example No. | Compound $R^5$ of Formula V | $ED_{50}$ (mg/kg)** | Lowest Active Dose (mg/kg) |
|---|---|---|---|
| 14 | 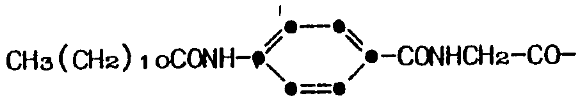 | >40 | >40 |
| 15 | 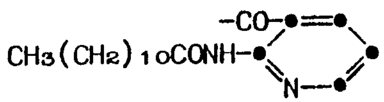 | >40 | >40 |
| 16 | $CH_3(CH_2)_5CONH(CH_2)_{10}-CO-$ | >40 | >40 |
| 17 | 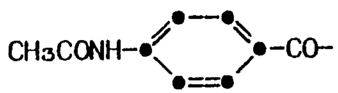 | >40 | >40 |
| 18 | 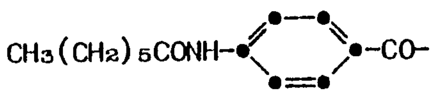 | >40 | >40 |
| 19 | 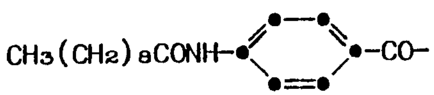 | 26 | 5 |

Table 7 (cont)

Therapeutic Activity Against Candida Albicans A-26 in Mice*

| Example No. | Compound $R^5$ of Formula V | $ED_{50}$ (mg/kg)** | Lowest Active Dose (mg/kg) |
|---|---|---|---|
| 20 | 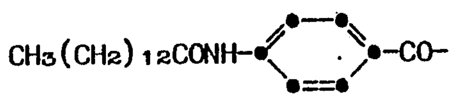 | 11 | 2.5 |
| 21 | 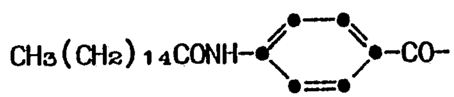 | 7 | 5 |
| 26 | 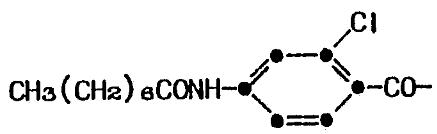 | 29 | 10 |
| 28 | 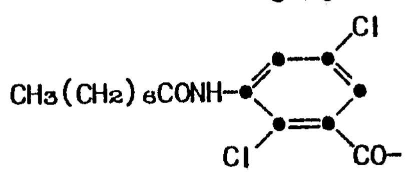 | >40 | 20 |

*Dosage Schedule: 40, 20, 15, and 10 mg/kg. Dosages given 0, 4, and 24 hours post injection as suspension of test compound in 30% PEG-$H_2O$. Number of mice receiving test compounds at each dosage level: 6 mice per group. Number of mice in control (untreated) group: 10 mice per group.

**As measured by increase in survival time of treated animals versus control, calculated by method of Reed V. Mueuch, American J. Hygiene, 493 (1938).

Table 8

Blood Levels after Administration in Mice

| Example No. | Compound: $R^5$ in Formula V | Blood Levels * (μg/ml) |
|---|---|---|
| 1 | $CH_3(CH_2)_{10}CONHCH(CH_2C_6H_5)-CO-$ | 0 |
| 2 | $CH_3(CH_2)_{10}CONH(CH_2)_4-CO-$ | 0 |
| 3 | $CH_3(CH_2)_{10}CONH(CH_2)_{10}-CO-$ | 0.40(0) |
| 4 | $CH_3(CH_2)_{10}CONH-$ 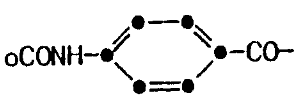 | 0.83 |
| 5 | $CH_3(CH_2)_{10}CONH-$ 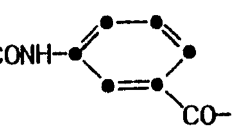 | 0 |
| 6 | $CH_3(CH_2)_{10}CONH-$ 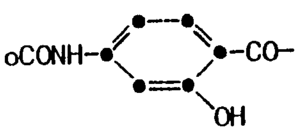 | 0 |
| 7 | $CH_3(CH_2)_{10}CONH-$ 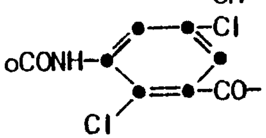 | 0.53 |

Table 8 (cont)

Blood Levels after Administration in Mice

| Example No. | Compound R5 in Formula V | Blood Levels * (μg/ml) |
|---|---|---|
| 8 | 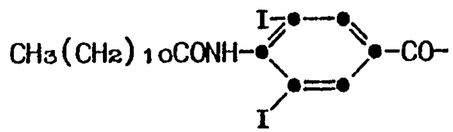 | 0.34 |
| 9 | 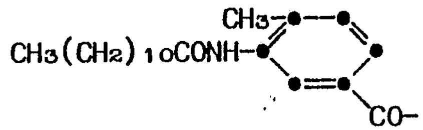 | 0 |
| 10 | 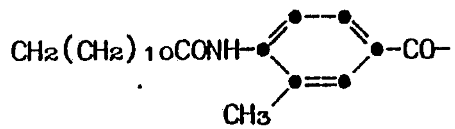 | 0 |
| 11 | 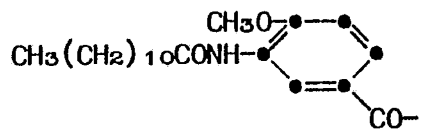 | -- |
| 12 | 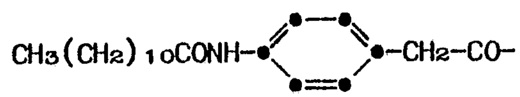 | 0.34 |
| 13 | 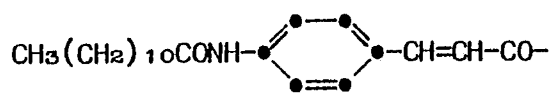 | 1.31 |

Table 8 (cont)

Blood Levels after Administration in Mice

| Example No. | Compound $R^5$ in Formula V | Blood Levels * (µg/ml) |
|---|---|---|
| 14 | 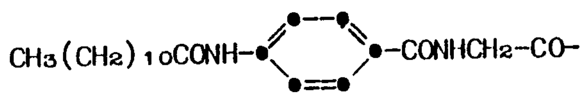 | 0.64 |
| 15 | 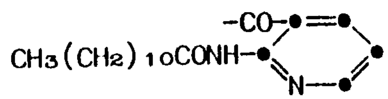 | 0 |
| 16 | $CH_3(CH_2)_5CONH(CH_2)_{10}-CO-$ | 0 |
| 17 | 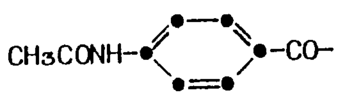 | 0 |
| 18 | 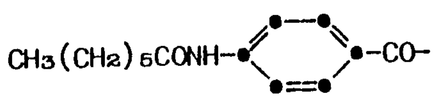 | -- |
| 19 | 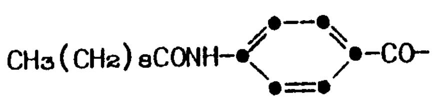 | -- |
| 20 | 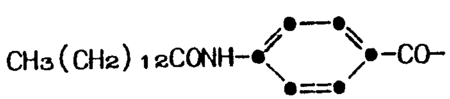 | 6.5 |

Table 8 (cont)

Blood Levels after Administration in Mice

| | Compound | |
|---|---|---|
| Example No. | $R^5$ in Formula V | Blood Levels * (μg/ml) |
| 21 | $CH_3(CH_2)_{14}CONH-$ 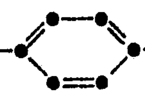 $-CO-$ | 36 |

*Four hours after administration of test compound at dose of 416 mg/kg by gavage as suspension of compound in 33% PEG 400-$H_2O$). Compound determined by bioassay vs. *Aspergillus montevidensis* A-35137.

CLAIMS

1. A compound of Formula:

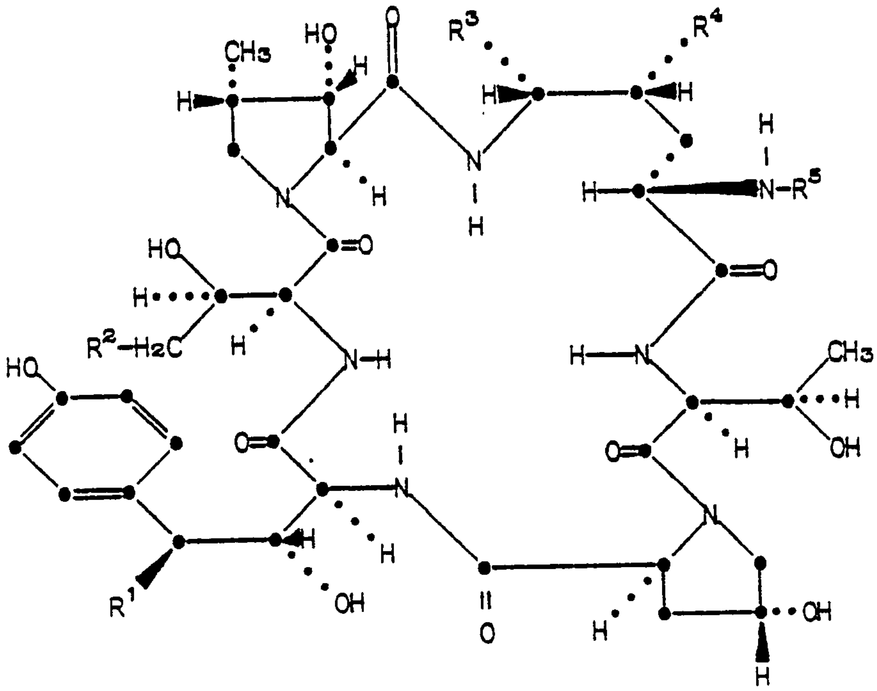

III

wherein $R^1$ is H or OH and;

when $R^1$ is H, $R^2$ is H and $R^3$ and $R^4$ are both H or both OH,

and

when $R^1$ is OH, $R^2$ is H, $R^3$ is OH or $C_1$-$C_6$ alkyloxy and $R^4$ is OH, or $R^2$ is -CO-$NH_2$ and $R^3$ and $R^4$ are both OH;

$R^5$ is an N-alkanoyl amino acyl group of the formula $-W-\overset{O}{\overset{\|}{C}}-R^6$ wherein:

W is a divalent aminoacyl radical of the formula:

(a) $-\overset{O}{\overset{\|}{C}}-A-NH-$

wherein A is $C_1$-$C_{10}$ alkylene or $C_5$-$C_6$ cycloalkylene;

(b) $-\overset{O}{\overset{\|}{C}}-\overset{R^7}{\overset{|}{C}}H-NH-$

wherein $R^7$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$-$C_3$ alkyl, hydroxy, $C_1$-$C_3$ alkylthio, carbamyl, or $C_1$-$C_3$ alkylcarbamyl;

(c)

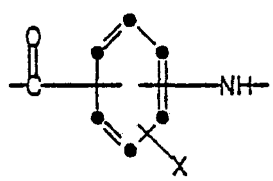

wherein X is hydrogen chloro, bromo, iodo, nitro, $C_1$-$C_3$ alkyl, hydroxy, $C_1$-$C_3$ alkoxy, mercapto, $C_1$-$C_3$ alkylthio, carbamyl, or $C_1$-$C_3$ alkylcarbamyl;

(d) 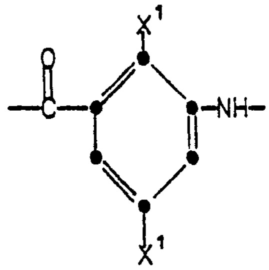 or 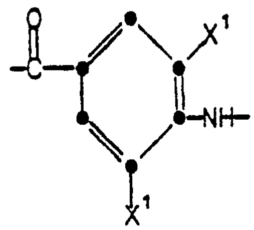

wherein $X^1$ is chloro, bromo, or iodo;

(e) 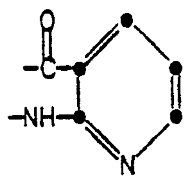 or 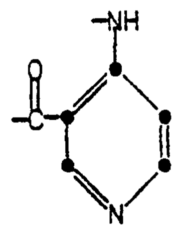 ; or

(f) 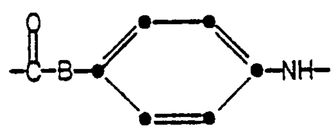

wherein B is a divalent radical of the formula: $-(CH_2)_n-$, wherein n is an integer from 1 to 3; $-CH=CH-$; $-CH=CH-CH_2-$; or

$$-\overset{O}{\overset{\|}{C}}NHCH_2-$$

and $R^6$ is $C_1$-$C_{17}$ alkyl or $C_2$-$C_{17}$ alkenyl.

2. A compound as defined in claim 1 wherein $R^5$ is $-\overset{O}{\overset{\|}{C}}-A-NH-\overset{O}{\overset{\|}{C}}R^6$ wherein A is $C_1$-$C_{10}$ alkylene and $R^6$ is straight chain $C_1$-$C_{17}$ alkyl.

3. The compound as defined in claim 2 wherein $R^5$ is N-(n-dodecanoyl)-5-amino-n-pentanoyl, N-(n-dodecanoyl)-11-amino-n-hendecanoyl, or N-(n-heptanoyl)-11-amino-n-hendecanoyl.

4. A compound as defined in claim 1 wherein $R^5$ is

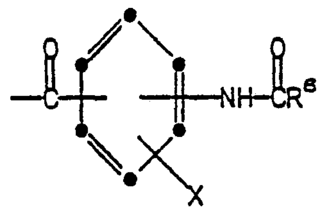

wherein X is hydrogen and $R^6$ is straight chain $C_1$-$C_{17}$ alkyl.

5. The compound as defined in claim 4 wherein $R^5$ is N-(n-dodecanoyl)-p-aminobenzoyl, N-(n-dodecanoyl)-m-aminobenzoyl, N-(acetyl)-p-aminobenzoyl, N-(n-heptanoyl)-p-aminobenzoyl, N-(n-decanoyl)-p-aminobenzoyl, N-(n-tetradecanoyl)-p-aminobenzoyl, or N-(n-hexadecanoyl)-p-aminobenzoyl.

6. A compound as defined in claim 1 wherein $R^5$ is

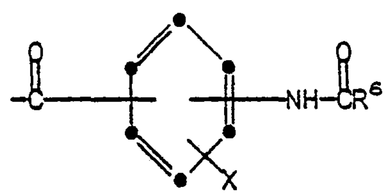

wherein X is chloro, bromo, iodo, nitro, $C_1$-$C_3$ alkyl, hydroxy, $C_1$-$C_3$ alkoxy, mercapto, $C_1$-$C_3$ alkylthio, carbamyl, or $C_1$-$C_3$ alkylcarbamyl, and $R^6$ is straight chain $C_1$-$C_{17}$ alkyl.

7. The compound as defined in claim 6 wherein $R^5$ is N-(n-dodecanoyl)-4-amino-2-hydroxybenzoyl, N-(n-dodecanoyl)-3-amino-4-methylbenzoyl, N-(n-dodecanoyl)-4-amino-3-methylbenzoyl, or N-(n-dodecanoyl)-3-amino-4-methoxybenzoyl.

8. The compound as defined in claim 1 wherein $R^5$ is N-(n-dodecanoyl)-3-amino-2,5-dichlorobenzoyl, N-(n-dodecanoyl)-4-amino-3,5-diodobenzoyl, N-(n-dodecanoyl)-p-aminophenylacetyl, N-(n-dodecanoyl)-p-aminocinnamoyl, N-(n-dodecanoyl)-p-aminohippuryl, N-(n-dodecanoyl)-2-aminonicotinyl, or N-(n-dodecanoyl)-phenylalanyl.

9. A process for the preparation of a compound of Formula III as defined in any of claims 1-8 which comprises acylating a cyclic peptide nucleus of Formula

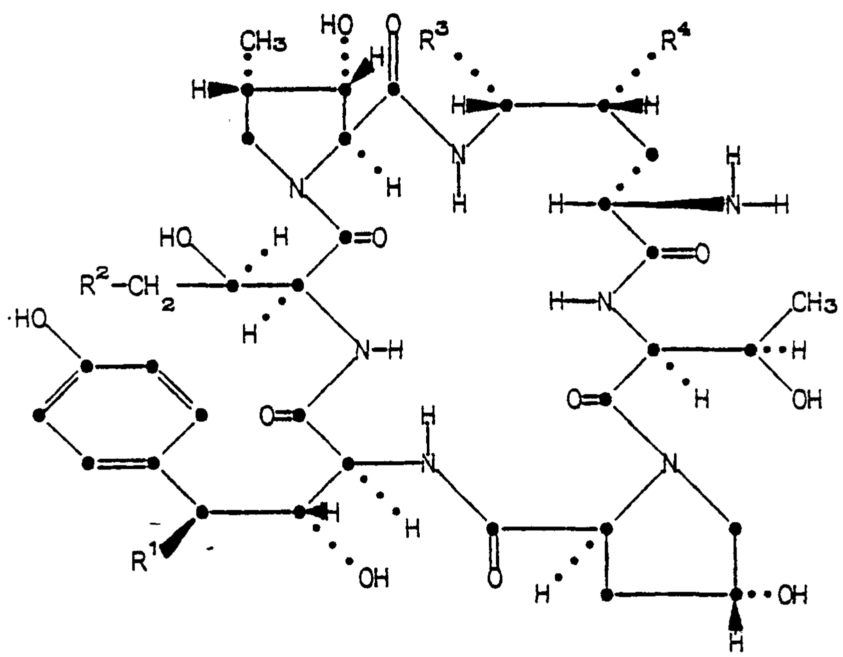

II

wherein $R^1$ is H or OH and;

when $R^1$ is H, $R^2$ is H and $R^3$ and $R^4$ are both H or both OH,

and

when $R^1$ is OH, $R^2$ is H, $R^3$ is OH or $C_1$-$C_6$ alkyloxy and $R^4$ is OH, or $R^2$ is $-\overset{O}{\overset{\|}{C}}-NH_2$ and $R^3$ and $R^4$ are both OH,

with an $R^5$ introducing acylating agent.

10. A process as defined in claim 9, wherein $R^5$ is an N-alkanoyl amino acyl group, derived from the corresponding acid of the formula $-W-\overset{O}{\overset{\|}{C}}-R^6$ wherein:

W is a divalent aminoacyl radical of the formula:

(a) $-\overset{O}{\overset{\|}{C}}-A-NH-$

wherein A is $C_1$-$C_{10}$ alkylene or $C_5$-$C_6$ cycloalkylene;

(b) $-\overset{O}{\overset{\|}{C}}-\overset{R^7}{\overset{|}{C}}H-NH-$

wherein $R^7$ is hydroxymethyl, hydroxyethyl, mercaptomethyl, mercaptoethyl, methylthioethyl, 2-thienyl, 3-indole-methyl, phenyl, benzyl, or substituted phenyl or substituted benzyl in which the benzene ring thereof is substituted with chloro, bromo, iodo, nitro, $C_1$-$C_3$ alkyl, hydroxy, $C_1$-$C_3$ alkylthio, carbamyl, or $C_1$-$C_3$ alkylcarbamyl;

(c) 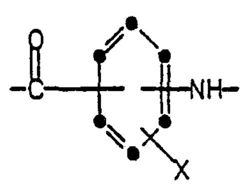

wherein X is hydrogen, chloro, bromo, iodo, nitro, $C_1$-$C_3$ alkyl, hydroxy, $C_1$-$C_3$ alkoxy, mercapto, $C_1$-$C_3$ alkylthio, carbamyl, or $C_1$-$C_3$ alkylcarbamyl;

(d) 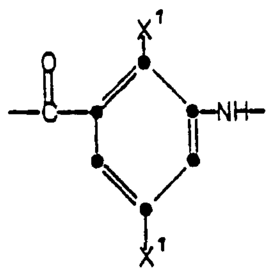 or 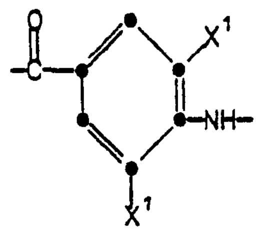

wherein $X^1$ is chloro, bromo, or iodo;

(e) 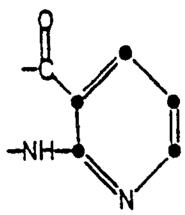 or 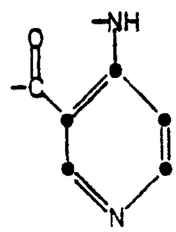 ; or

(f) 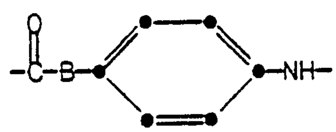

wherein B is a divalent radical of the formula: $-(CH_2)_n-$, wherein n is an integer from 1 to 3; $-CH=CH-$; $-CH=CH-CH_2-$; or

$-\overset{O}{\overset{\parallel}{C}}NHCH_2-$

and $R^6$ is $C_1$-$C_{17}$ alkyl or $C_2$-$C_{17}$ alkenyl.

0032009

European Patent Office

# EUROPEAN SEARCH REPORT

Application number
EP 80 30 4471

| Category | Citation of document with indication, where appropriate, of relevant passages | Relevant to claim |
|---|---|---|
| | CHEMICAL ABSTRACTS, vol. 91, no. 13, September 24, 1979, page 608, abstract 108214r COLUMBUS, Ohio (US) & Helv. Chim. Acta 1979, 62(4), 1252-56 R. TRABER et al.:"Cyclopeptide antibiotics from Aspergillus species. Structure of echinocandins C and D" * the whole abstract * -- | 1 |
| | DE - A - 2 704 030 (SANDOZ) * the whole document * -- | 1,2 |
| | DE - A - 2 742 435 (SANDOZ) * the whole document * -- | 1,2 |
| | DE - A - 2 803 584 (SANDOZ) * the whole document * -- | 1,2 |
| P | BE - A - 883 593 (ELI LILLY) * the whole document * & EP - A - 0 021 685 -- | 1,2 |
| D | US - A - 4 024 245 (ELI LILLY) * the whole document * ------ | 1,2 |

CLASSIFICATION OF THE APPLICATION (Int. Cl.3)

A 61 K 37/02
C 07 C 103/52
C 12 P 21/04
//C 12 P 21/04
C 12 R 1/045
1/62)

TECHNICAL FIELDS SEARCHED (Int. Cl.3)

C 07 C 103/52
C 12 P 21/04

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 25-03-1981 | RAJIC |